# EUROPEAN PATENT APPLICATION

(11) **EP 4 242 233 A1**
(43) Date of publication of application: **13.09.2023**
(21) Application number: 21889549.8
(22) Date of filing: 03.11.2021
(51) Int. Cl.: C07K 16/28, A61P 35/00, A61P 37/00, A61P 25/28

(54) **FC ALPHA RECEPTOR BINDING ANTIBODY**

(30) Priority: 06.11.2020 KR 20200147590; 06.11.2020 KR 20200147716; 06.11.2020 KR 20200147845; 12.10.2021 KR 20210135094; 12.10.2021 KR 20210135095
(71) Applicant: Korea University Research and Business Foundation, Seoul 02841 (KR)
(72) Inventor: JUNG, Sang Taek, Seoul 06217 (KR); LEE, Ji Sun, Seoul 03472 (KR); KIM, Bo Mi, Suwon-si Gyeonggi-do 16316 (KR)
(74) Representative: Isarpatent
(86) International application number: PCT/KR2021/015795
(87) International publication number: WO 2022/098084

(57) **Abstract**

The present invention relates to an antibody having enhanced binding affinity for human Fc alpha receptor and a technique for maximizing a mechanism of antibody action by using same. Antibodies specific for Fc alpha receptors or immunologically active fragments thereof according to the present invention are in the form of IgG and bind to Fc alpha receptors of effector cells, especially, neutrophils, which are most abundant in mammals, thereby overcoming the disadvantages of conventional IgA antibodies and maximizing an effector function in various antibody therapeutic agents, which leads to maximizing the mechanism of antibody action (ADCC and ADCP). Thus, various Fc-fused protein therapeutic agents using same can be advantageously utilized as antibody drugs with enhanced effector functions.

## Description

### [Technical Field]

The present invention relates to an antibody having enhanced binding affinity for a human Fc alpha receptor and a technique for maximizing a mechanism of antibody action by using the same.

### [Background Art]

An antibody provides a linkage between the humoral and cellular immune systems, and a Fab region of the antibody recognizes an antigen, whereas an Fc region binds to an Fc receptor (FcR) that is differentially expressed by all immune competent cells. The cross-linking of receptors by multivalent antigen/antibody complexes triggers degranulation, cytolysis or phagocytosis of target cells and transcription-activation of cytokine-encoding genes (Deo, Y.M. et al., Immunol. Today 19(3):127-135 (1997)). The antibody binds to a cell through the Fc region by binding an Fc receptor binding site on the antibody Fc region to an Fc receptor (FcR) on the cell. The receptors for the Fc region of immunoglobulin are important in triggering many protective functions of monocytes, macrophages and polymorphonuclear cells. Receptors (Fc receptors or FcRs) for immunoglobulins on these cells have been extensively studied, and monoclonal antibodies for these receptors have been generated and found to be therapeutically effective. The antibody binds to the Fc receptor on the cell surface to trigger many important and diverse biological responses including phagocytosis and destruction of antibody-coated particles, removal of immune complexes, lysis of antibody-coated target cells by killer cells (known as antibody-dependent cell-mediated cytotoxicity, or ADCC), release of inflammatory mediators, placental migration and control of immunoglobulin production.

There are several Fc receptors specific for different types of antibodies including IgG (λ receptor), IgE (ε receptor), IgA (α receptor) and IgM (µ receptor). Among them, the IgA receptor (Fcα receptor or CD89) may enhance the function of effector cells. The ligand binds to the Fcα receptor to trigger antibody-dependent cellular phagocytosis (ADCP) and antibody-dependent cellular cytotoxicity (ADCC) of leukocytes and Fcα receptor-bearing cell lines. In addition, the Fcα receptor may enhance phagocytosis on target cells in cooperation with the receptor for IgG on effector cells. The effector function mediated by the antibody Fc region may be divided into two categories: (1) effector functions that operate after an antibody binds to an antigen (such functions include, for example, involvement in complement cascade amplification or Fc receptor (FcR)-bearing cells); and (2) effector functions that operate separately from antigen binding (these functions confer persistence in circulation and the ability to migrate across cell barriers, for example, by transcytosis). For example, a complement system is activated when a Cl component of the complement binds to the antibody. The activation of the complement is important for the opsonization and lysis of cellular pathogens. The activation of the complement also stimulates the inflammatory response and may be involved in autoimmune hypersensitivity. As such, the Fcα receptor or CD89 is a receptor that binds to the Fc region of IgA (Kerr, M. A. 1990, Biochem. J. 271: 285-296), and is constitutively expressed on cytotoxic immune effector cells, mainly including polymorphonuclear leukocytes (PMN), monocytes, macrophages, neutrophils and eosinophils (Morton, H. C., et al., 1996, Critical Reviews in Immunology 16: 423). In addition, it was reported that the Fcα receptor is expressed on a lymphocyte subpopulation (Morton, H. C., et al., 1996, Critical Reviews in Immunology 16: 423), and expressed even on glomerular mesangial cells (Gomez-Guerrero, C., et al., 1996, J. Immunol. 156: 4369-4376). A lot of α-chains of the human Fcα receptor are glycosylated and type I transmembrane molecules belonging to an Ig super-gene group including receptors for IgG and IgE. One gene located on chromosome 19 encodes a plurality of variably spliced isotypes of an FcαRI alpha chain (55 to 110 kDa; Morton, H. C., et al., 1996, Critical Reviews in Immunology 16: 423). Myeloid Fcoc receptors have been found to be associated with an FcR γ-chain, which plays a role in Fcα receptor signaling (Morton, H. C. et al. 1995, J. Biol. Chem. 270: 29781; Pfefferkom, L. C., et al. 1995, J. Immunol. 153: 3228-3236; Saito, K. et al., 1995, J. Allergy Clin. Immunol. 96: 1152). The Fcoc receptors bind to antigen-bound IgAl and IgA2 and monomeric IgAl and IgA2 (Mazangera, R. L. et al., 1990, Biochem. J. 272: 159-165), which is consistent with the saturation of the receptor with a monomeric IgA antigen in vivo in the same manner as the saturation of FcyR and FcεRI with IgG and IgE, respectively. Cross-linking of mAbs specific for polymeric IgA, IgA immune complexes, or epitopes inside or outside a ligand binding domain with Fcoc receptors on bone marrow effector cells stimulates degranulation, superoxide release, secretion of inflammatory cytokines, endocytosis and phagocytosis (Patty, C.,A. Herbelin, A. Lihuen, J. F. Bach, and R. C. Monteiro, 1995, Immunology 86: 1-5; Stewart, W. W., R. L. MazYegera, L. Shen, and M. A. Kerr, 1994, J. Leucocyte Biology 56: 481-487; Stewart, W. W., and M. A. Kerr, 1990, Immunology 71: 328-334; Shen, L., 1992, J. Leukocyte Biology 51: 373-378). These physiological responses triggered by the Fcα receptor may be important for first-line humoral defense on mucosal surfaces (Morton, H. C., M. van Egmond, and J. G. J. van de Winkel, 1996, Critical Reviews in Immunology 16: 423).

Meanwhile, a plurality of research results suggest that an Fc-receptor-dependent mechanism is a substantial cause for the action of cytotoxic antibodies on tumors, and it was shown that optimal antibodies against tumors bind preferentially to the activated Fc receptor (Clynes, R. A., et al., Nature Medicine 6(4):443-446 (2000); Kalergis, A. M., and Ravetch, J. V., J. Exp. Med. 195 (12):1653-1659 (June, 2002). Often, successful anti-cancer monoclonal antibodies activate cell signaling cascades other than ADCC or block access to growth factors to induce an Fc-independent direct signaling mechanism that regulates target cell survival, proliferation or cell death (Selenko, N., et al., J Clin. Immunol. 22 (3):124-130 (2002)). For example, it was confirmed that the CD20⁺ B cells are treated with rituximab to induce Mab-induced apoptosis and induce complement-mediated cytolysis and ADCC (Selenko, N., et al., J. Clin. Immunol. 22 (3):124-130 (2002)). In addition, rituximab-induced apoptosis of lymphoma cells not only kills the cells, but also promotes uptake and cross-presentation of lymphoma cell-derived peptides by antigen-presenting dendritic cells (DCs), induces maturation of DCs and generates specific cytotoxic T lymphocytes (CTLs).

As the Fc receptor, there are several Fc receptors specific for different types of antibodies including IgG (λ receptor), IgE (ε receptor), IgA (α receptor) and IgM (µ receptor). Among them, the IgA receptor (Fcα receptor or CD89) may enhance the function of effector cells. The ligand binds to the Fcα receptor to trigger antibody-dependent cellular phagocytosis (ADCP) and antibody-dependent cellular cytotoxicity (ADCC) of leukocytes and Fcα receptor-bearing cell lines. In addition, the Fcα receptor may enhance phagocytosis on target cells in cooperation with the receptor for IgG on effector cells. The effector function mediated by the antibody Fc region may be divided into two categories: (1) effector functions that operate after an antibody binds to an antigen (such functions include, for example, involvement in complement cascade amplification or Fc receptor (FcR)-bearing cells); and (2) effector functions that operate separately from antigen binding (these functions confer persistence in circulation and the ability to migrate across cell barriers, for example, by transcytosis). For example, a complement system is activated when a Cl component of the complement binds to the antibody. The activation of the complement is important for the opsonization and lysis of cellular pathogens. The activation of the complement also stimulates the inflammatory response and may be involved in autoimmune hypersensitivity. As such, the Fcα receptor or CD89 is a receptor that binds to the Fc region of IgA (Kerr, M. A. 1990, Biochem. J. 271: 285-296), and is constitutively expressed on cytotoxic immune effector cells, mainly including polymorphonuclear leukocytes (PMN), monocytes, macrophages, neutrophils and eosinophils (Morton, H. C., et al., 1996, Critical Reviews in Immunology 16: 423). In addition, it was reported that the Fcα receptor is expressed on a lymphocyte subpopulation (Morton, H. C., et al., 1996, Critical Reviews in Immunology 16: 423), and expressed even on glomerular mesangial cells (Gomez-Guerrero, C., et al., 1996, J. Immunol. 156: 4369-4376). A lot of α-chains of the human Fcα receptor are glycosylated and type I transmembrane molecules belonging to an Ig super-gene family including receptors for IgG and IgE. One gene located on chromosome 19 encodes a plurality of variably spliced isotypes of an FcαRI alpha chain (55 to 110 kDa; Morton, H. C., et al., 1996, Critical Reviews in Immunology 16: 423). Myeloid Fcα receptors have been found to be associated with an FcR γ-chain, which plays a role in Fcα receptor signaling (Morton, H. C. et al. 1995, J. Biol. Chem. 270: 29781; Pfefferkom, L. C., et al. 1995, J. Immunol. 153: 3228-3236; Saito, K. et al., 1995, J. Allergy Clin. Immunol. 96: 1152). The Fcα receptors bind to antigen-bound IgAl and IgA2 and monomeric IgAl and IgA2 (Mazangera, R. L. et al., 1990, Biochem. J. 272: 159-165), which is consistent with the saturation of the receptor with a monomeric IgA antigen in vivo in the same manner as the saturation of FcyR and FcεRI with IgG and IgE, respectively. Cross-linking of mAbs specific for polymeric IgA, IgA immune complexes, or epitopes inside or outside a ligand binding domain with Fcoc receptors on bone marrow effector cells stimulates degranulation, superoxide release, secretion of inflammatory cytokines, endocytosis and phagocytosis (Patty, C.,A. Herbelin, A. Lihuen, J. F. Bach, and R. C. Monteiro, 1995, Immunology 86: 1-5; Stewart, W. W., R. L. MazYegera, L. Shen, and M. A. Kerr, 1994, J. Leucocyte Biology 56: 481-487; Stewart, W. W., and M. A. Kerr, 1990, Immunology 71: 328-334; Shen, L., 1992, J. Leukocyte Biology 51: 373-378). These physiological responses triggered by the Fcα receptor may be important for first-line humoral defense on mucosal surfaces (Morton, H. C., M. van Egmond, and J. G. J. van de Winkel, 1996, Critical Reviews in Immunology 16: 423).

However, there is a very large disadvantage that IgA using the effector function of leukocytes, which has the highest rate in mammals, has significantly lower productivity than that of IgG due to complex glycosylation and tailpiece in addition to the presence of a dimer form and does not bind to FcRn, which is known to be involved in recycling of the antibody in the body to have a short half-life of 1 week as compared with IgG having a half-life of 3 weeks.

### [Disclosure]

### [Technical Problem]

An object of the present invention is to provide a novel IgG antibody binding to an Fc alpha receptor or an immunologically active fragment thereof, in order to improve the disadvantages of IgA antibodies having low productivity and short half-life, and to maximize an effector function of therapeutic protein drugs, and to present bispecific or multispecific antibody platforms in various forms to utilize the antibody as a therapeutic antibody.

### [Technical Solution]

In order to achieve the object, an aspect of the present invention provides an antibody specific for an Fc alpha receptor or an immunologically active fragment thereof.

Further, another aspect of the present invention provides a bispecific or multispecific antibody.

Further, yet another aspect of the present invention provides an isolated nucleic acid molecule encoding the antibody, a vector including the same, and a host cell transformed with the vector.

Further, still another aspect of the present invention provides a pharmaceutical composition including a bispecific or multispecific antibody as an active ingredient.

Further, still yet another aspect of the present invention provides a method for preparing an antibody specific for an Fc alpha receptor or an immunologically active fragment thereof.

In addition, still yet another aspect of the present invention provides a method for preparing a bispecific or multispecific antibody.

### [Advantageous Effects]

According to the present invention, antibodies specific for Fc alpha receptors or immunologically active fragments thereof are in the form of IgG and bind to Fc alpha receptors of effector cells, especially, neutrophils, which are most abundant in mammals, thereby overcoming the disadvantages of conventional IgA antibodies and maximizing an effector function in various antibody therapeutic agents, which leads to maximizing the mechanism of antibody action (ADCC and ADCP). Thus, various Fc-fused protein therapeutic agents using same may be advantageously utilized as antibody drugs with enhanced effector functions.

### [Description of Drawings]

FIG. 1 is a diagram illustrating prepared dimeric Fc alpha receptor-ECD region antigen protein and tetrameric Fc alpha receptor-ECD region antigen protein vectors and a result of SDS-PAGE analysis thereof.
FIG. 2 is a diagram illustrating an antibody search schematic diagram using a flow cytometer.
FIG. 3 is a diagram illustrating results of amino acid sequence analysis of six scFv human antibody variants showing high binding affinity to Fc alpha receptor-ECD.
FIG. 4 illustrates results of Fc alpha receptor binding affinity analysis of six scFv antibody variants.
FIG. 5 is a diagram illustrating vectors of IgG JS9, JS19, JS30, JS40, JS41 and JS48 each including six scFv antibody variants, and SDS-PAGE gel photographs after expression and purification.
FIG. 6 is a diagram of confirming the antigen binding affinity of IgG antibodies JS9, JS30, JS40, JS41 and JS48 each including six scFv antibody variants.
FIG. 7 is a diagram illustrating results of amino acid sequence analysis of three novel scFv human antibody variants showing increased binding affinity for Fc alpha receptor-ECD discovered by a high-speed search system.
FIG. 8 is a diagram illustrating the binding affinity for Fc alpha receptor-ECD of three novel scFv antibody variants JS 116, JS144 and JS140 with increased binding affinity for Fc alpha receptor-ECD by a flow cytometer.
FIG. 9 is a diagram illustrating animal cell expression vectors for preparing three novel scFv antibody variants with increased binding affinity for Fc alpha receptor-ECD in the form of IgG, and expression and purification of the vectors in animal cells by SDS-PAGE.
FIG. 10 is a diagram illustrating results of confirming the binding affinity for the Fc alpha receptor-ECD of three antibodies with increased binding affinity for the Fc alpha receptor-ECD prepared in the form of IgG by ELISA.
FIG. 11 is a diagram of animal cell expression vectors for producing a JS40-1 antibody having a VH region of JS40 and a VL region of JS48 and a JS48-1 antibody having a VL region of JS40 while having a VH region of JS48, and expression and purification of the vectors in animal cells by SDS-PAGE.
FIG. 12 is a diagram illustrating results of confirming the binding affinity for the Fc alpha receptor-ECD of the antibody JS40-1 and the antibody JS48-1 by ELISA.
FIG. 13 is a diagram illustrating expression vectors of bispecific antibodies (8 types) that bind to two types of antigens (Fc alpha receptor and Her-2) prepared using IgG antibodies JS40 and JS48 and trastuzumab.
FIG. 14 is a diagram showing SDS-PAGE gel photographs of 8 types of bispecific antibodies expressed and purified in animal cells.
FIG. 15 is a diagram of confirming the binding affinity for two antigens of bispecific antibodies.
A and B: Binding affinity for Fc alpha receptor; and
C and D: Binding affinity for Her-2

### [Best Mode of the Invention]

Hereinafter, an exemplary embodiment of the present invention will be described in detail with reference to the accompanying drawings. However, the following exemplary embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

In addition, terminologies used herein are terminologies used to properly express examples of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned herein as abbreviations are also described as follows according to the IUPAC-IUB nomenclature.

Alanine: A, Arginine: R, Asparagine: N, Aspartic acid: D, Cysteine: C, Glutamic acid: E, Glutamine: Q, Glycine: G, Histidine: H, Isoleucine: I, Leucine: L, Lysine: K, Methionine: M, Phenylalanine: F, Proline: P, Serine: S, Threonine: T, Tryptophan: W, Tyrosine: Y, and Valine: V.

In one aspect, the present invention relates to an antibody specific for an Fc alpha receptor or an immunologically active fragment thereof.

In an exemplary embodiment, the antibody may be IgG, and the immunologically active fragment may be any one selected from the group consisting of Fab, Fd, Fab', dAb, F(ab'), F(ab')₂, a single chain fragment variable (scFv), Fv, a single chain antibody, a Fv dimer, a complementarity determining region (CDR) fragment, a humanized antibody, a chimeric antibody, and a diabody, and more preferably scFv.

In an exemplary embodiment, the antibody specific for the Fc alpha receptor or the immunologically active fragment thereof of the present invention may include (a) a VH domain including complementarity determining region heavy chain (CDRH)1 including any one selected from the group consisting of amino acid sequences of SEQ ID NOs: 1 to 3, CDRH2 including any one selected from the group consisting of amino acid sequences of SEQ ID NOs: 4 to 7, and CDRH3 including any one selected from the group consisting of amino acid sequences of SEQ ID NOs: 8 to 15; and/or (b) a VL domain including complementarity determining region light chain (CDRL)1 including any one selected from the group consisting of amino acid sequences of SEQ ID NOs: 16 to 21, CDRL2 including any one selected from the group consisting of amino acid sequences of SEQ ID NOs: 22 to 27, and CDRL3 including any one selected from the group consisting of amino acid sequences of SEQ ID NOs: 28 to 32.

In an exemplary embodiment, the antibody specific for the Fc alpha receptor or the immunologically active fragment thereof of the present invention may include (a) a VH domain including FR1 including any one selected from the group consisting of amino acid sequences of SEQ ID NOs: 33 to 36, FR2 including any one selected from the group consisting of amino acid sequences of SEQ ID NOs: 37 to 41, FR3 including any one selected from the group consisting of amino acid sequences of SEQ ID NOs: 42 to 44, and FR4 including an amino acid sequence of SEQ ID NO: 45 or 46; and/or (b) a VL domain including FR1 including any one selected from the group consisting of amino acid sequences of SEQ ID NOs: 47 to 52, FR2 including any one selected from the group consisting of amino acid sequences of SEQ ID NOs: 53 to 56, FR3 including any one selected from the group consisting of amino acid sequences of SEQ ID NOs: 57 to 63, and FR4 including any one selected from the group consisting of amino acid sequences of SEQ ID NOs: 64 to 67.

In an exemplary embodiment, the antibody specific for the Fc alpha receptor or the immunologically active fragment thereof of the present invention may be JS40 including a V domain including (a) a VH domain including FR1 including an amino acid sequence of SEQ ID NO: 33, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 11, and FR4 including an amino acid sequence of SEQ ID NO: 46; and/or (b) a VL domain including FR1 including an amino acid sequence of SEQ ID NO: 47, CDRL1 including an amino acid sequence of SEQ ID NO: 16, FR2 including an amino acid sequence of SEQ ID NO: 53, CDRL2 including an amino acid sequence of SEQ ID NO: 24, FR3 including an amino acid sequence of SEQ ID NO: 57, CDRL3 including an amino acid sequence of SEQ ID NO: 28, and FR4 including an amino acid sequence of SEQ ID NO: 64.

In an exemplary embodiment, the antibody specific for the Fc alpha receptor or the immunologically active fragment thereof of the present invention may be JS48 including a V domain including (a) a VH domain including FR1 including an amino acid sequence of SEQ ID NO: 34, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 11, and FR4 including an amino acid sequence of SEQ ID NO: 46; and/or (b) a VL domain including FR1 including an amino acid sequence of SEQ ID NO: 49, CDRL1 including an amino acid sequence of SEQ ID NO: 17, FR2 including an amino acid sequence of SEQ ID NO: 54, CDRL2 including an amino acid sequence of SEQ ID NO: 23, FR3 including an amino acid sequence of SEQ ID NO: 59, CDRL3 including an amino acid sequence of SEQ ID NO: 30, and FR4 including an amino acid sequence of SEQ ID NO: 65.

In an exemplary embodiment, the antibody specific for the Fc alpha receptor or the immunologically active fragment thereof of the present invention may be JS 116 including a V domain including (a) a VH domain including FR1 including an amino acid sequence of SEQ ID NO: 33, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 8, and FR4 including an amino acid sequence of SEQ ID NO: 45; and/or (b) a VL domain including FR1 including an amino acid sequence of SEQ ID NO: 47, CDRL1 including an amino acid sequence of SEQ ID NO: 16, FR2 including an amino acid sequence of SEQ ID NO: 53, CDRL2 including an amino acid sequence of SEQ ID NO: 22, FR3 including an amino acid sequence of SEQ ID NO: 57, CDRL3 including an amino acid sequence of SEQ ID NO: 28, and FR4 including an amino acid sequence of SEQ ID NO: 64.

In an exemplary embodiment, the antibody specific for the Fc alpha receptor or the immunologically active fragment thereof of the present invention may be JS 144 including a V domain including (a) a VH domain including FR1 including an amino acid sequence of SEQ ID NO: 33, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 9, and FR4 including an amino acid sequence of SEQ ID NO: 45; and/or (b) a VL domain including FR1 including an amino acid sequence of SEQ ID NO: 47, CDRL1 including an amino acid sequence of SEQ ID NO: 16, FR2 including an amino acid sequence of SEQ ID NO: 53, CDRL2 including an amino acid sequence of SEQ ID NO: 22, FR3 including an amino acid sequence of SEQ ID NO: 57, CDRL3 including an amino acid sequence of SEQ ID NO: 28, and FR4 including an amino acid sequence of SEQ ID NO: 64.

In an exemplary embodiment, the antibody specific for the Fc alpha receptor or the immunologically active fragment thereof of the present invention may be JS 140 including a V domain including (a) a VH domain including FR1 including an amino acid sequence of SEQ ID NO: 34, CDRH1 including an amino acid sequence of SEQ ID NO: 1, FR2 including an amino acid sequence of SEQ ID NO: 38, CDRH2 including an amino acid sequence of SEQ ID NO: 4, FR3 including an amino acid sequence of SEQ ID NO: 42, CDRH3 including an amino acid sequence of SEQ ID NO: 10, and FR4 including an amino acid sequence of SEQ ID NO: 46; and/or (b) a VL domain including FR1 including an amino acid sequence of SEQ ID NO: 48, CDRL1 including an amino acid sequence of SEQ ID NO: 17, FR2 including an amino acid sequence of SEQ ID NO: 54, CDRL2 including an amino acid sequence of SEQ ID NO: 23, FR3 including an amino acid sequence of SEQ ID NO: 58, CDRL3 including an amino acid sequence of SEQ ID NO: 29, and FR4 including an amino acid sequence of SEQ ID NO: 65.

In an exemplary embodiment, the antibody specific for the Fc alpha receptor or the immunologically active fragment thereof of the present invention may be JS40-1 including a V domain including (a) a VH domain including FR1 including an amino acid sequence of SEQ ID NO: 34, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 11, and FR4 including an amino acid sequence of SEQ ID NO: 46; and/or (b) a VL domain including FR1 including an amino acid sequence of SEQ ID NO: 49, CDRL1 including an amino acid sequence of SEQ ID NO: 16, FR2 including an amino acid sequence of SEQ ID NO: 54, CDRL2 including an amino acid sequence of SEQ ID NO: 24, FR3 including an amino acid sequence of SEQ ID NO: 59, CDRL3 including an amino acid sequence of SEQ ID NO: 28, and FR4 including an amino acid sequence of SEQ ID NO: 65.

In an exemplary embodiment, the antibody specific for the Fc alpha receptor or the immunologically active fragment thereof of the present invention may be JS40-1 including a V domain including (a) a VH domain including FR1 including an amino acid sequence of SEQ ID NO: 33, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 11, and FR4 including an amino acid sequence of SEQ ID NO: 45; and/or (b) a VL domain including FR1 including an amino acid sequence of SEQ ID NO: 47, CDRL1 including an amino acid sequence of SEQ ID NO: 17, FR2 including an amino acid sequence of SEQ ID NO: 53, CDRL2 including an amino acid sequence of SEQ ID NO: 23, FR3 including an amino acid sequence of SEQ ID NO: 57, CDRL3 including an amino acid sequence of SEQ ID NO: 30, and FR4 including an amino acid sequence of SEQ ID NO: 64.

In an exemplary embodiment, the antibody specific for the Fc alpha receptor or the immunologically active fragment thereof of the present invention may be JS9 including a V domain including (a) a VH domain including FR1 including an amino acid sequence of SEQ ID NO: 35, CDRH1 including an amino acid sequence of SEQ ID NO: 2, FR2 including an amino acid sequence of SEQ ID NO: 39, CDRH2 including an amino acid sequence of SEQ ID NO: 6, FR3 including an amino acid sequence of SEQ ID NO: 43, CDRH3 including an amino acid sequence of SEQ ID NO: 23, and FR4 including an amino acid sequence of SEQ ID NO: 46; and/or (b) a VL domain including FR1 including an amino acid sequence of SEQ ID NO: 50, CDRL1 including an amino acid sequence of SEQ ID NO: 18, FR2 including an amino acid sequence of SEQ ID NO: 55, CDRL2 including an amino acid sequence of SEQ ID NO: 25, FR3 including an amino acid sequence of SEQ ID NO: 60, CDRL3 including an amino acid sequence of SEQ ID NO: 31, and FR4 including an amino acid sequence of SEQ ID NO: 66.

In an exemplary embodiment, the antibody specific for the Fc alpha receptor or the immunologically active fragment thereof of the present invention may be JS19 including a V domain including (a) a VH domain including FR1 including an amino acid sequence of SEQ ID NO: 33, CDRH1 including an amino acid sequence of SEQ ID NO: 1, FR2 including an amino acid sequence of SEQ ID NO: 41, CDRH2 including an amino acid sequence of SEQ ID NO: 4, FR3 including an amino acid sequence of SEQ ID NO: 42, CDRH3 including an amino acid sequence of SEQ ID NO: 15, and FR4 including an amino acid sequence of SEQ ID NO: 46; and/or (b) a VL domain including FR1 including an amino acid sequence of SEQ ID NO: 52, CDRL1 including an amino acid sequence of SEQ ID NO: 21, FR2 including an amino acid sequence of SEQ ID NO: 56, CDRL2 including an amino acid sequence of SEQ ID NO: 27, FR3 including an amino acid sequence of SEQ ID NO: 63, CDRL3 including an amino acid sequence of SEQ ID NO: 32, and FR4 including an amino acid sequence of SEQ ID NO: 67.

In an exemplary embodiment, the antibody specific for the Fc alpha receptor or the immunologically active fragment thereof of the present invention may be JS30 including a V domain including (a) a VH domain including FR1 including an amino acid sequence of SEQ ID NO: 36, CDRH1 including an amino acid sequence of SEQ ID NO: 3, FR2 including an amino acid sequence of SEQ ID NO: 40, CDRH2 including an amino acid sequence of SEQ ID NO: 7, FR3 including an amino acid sequence of SEQ ID NO: 44, CDRH3 including an amino acid sequence of SEQ ID NO: 13, and FR4 including an amino acid sequence of SEQ ID NO: 46; and/or (b) a VL domain including FR1 including an amino acid sequence of SEQ ID NO: 51, CDRL1 including an amino acid sequence of SEQ ID NO: 19, FR2 including an amino acid sequence of SEQ ID NO: 54, CDRL2 including an amino acid sequence of SEQ ID NO: 26, FR3 including an amino acid sequence of SEQ ID NO: 61, CDRL3 including an amino acid sequence of SEQ ID NO: 30, and FR4 including an amino acid sequence of SEQ ID NO: 65.

In an exemplary embodiment, the antibody specific for the Fc alpha receptor or the immunologically active fragment thereof of the present invention may be JS41 including a V domain including (a) a VH domain including FR1 including an amino acid sequence of SEQ ID NO: 36, CDRH1 including an amino acid sequence of SEQ ID NO: 3, FR2 including an amino acid sequence of SEQ ID NO: 40, CDRH2 including an amino acid sequence of SEQ ID NO: 7, FR3 including an amino acid sequence of SEQ ID NO: 44, CDRH3 including an amino acid sequence of SEQ ID NO: 14, and FR4 including an amino acid sequence of SEQ ID NO: 46; and/or (b) a VL domain including FR1 including an amino acid sequence of SEQ ID NO: 51, CDRL1 including an amino acid sequence of SEQ ID NO: 20, FR2 including an amino acid sequence of SEQ ID NO: 54, CDRL2 including an amino acid sequence of SEQ ID NO: 23, FR3 including an amino acid sequence of SEQ ID NO: 62, CDRL3 including an amino acid sequence of SEQ ID NO: 30, and FR4 including an amino acid sequence of SEQ ID NO: 65.

In an exemplary embodiment, the antibody specific for the Fc alpha receptor or immunologically active fragment thereof of the present invention may include CL including an amino acid sequence of SEQ ID NO: 68, CH1 including an amino acid sequence of SEQ ID NO: 69, CH2 including an amino acid sequence of SEQ ID NO: 70 or CH3 including an amino acid sequence of SEQ ID NO: 71.

In an exemplary embodiment, the antibody specific for the Fc alpha receptor or immunologically active fragment thereof of the present invention may specifically bind to an extra cellular domain (ECD) of the Fc alpha receptor, and the ECD of the Fc alpha receptor may include an amino acid sequence of SEQ ID NO: 72.

In an exemplary embodiment, the antibody specific for the Fc alpha receptor or immunologically active fragment thereof of the present invention may increase an effector function and may increase antibody-dependent cellular phagocytosis (ADCP) or antibody-dependent cellular cytotoxicity (ADCC) of leukocytes and CD89-bearing cell lines.

In the present invention, the term "Fc alpha receptor (Fcα receptor)" may also be used interchangeably with an IgA receptor or CD89.

The antibody includes functional fragments of an antibody molecule as well as a whole antibody form. The whole antibody has a structure having two full-length light chains and two full-length heavy chains, and each light chain is linked to the heavy chain by disulfide bonds. A functional fragment of the antibody molecule refers to a fragment having an antigen-binding function, and examples of the functional fragment include (a) a Fab fragment consisting of a light chain variable region VL, a heavy chain variable region VH, a light chain constant region CL, and a first heavy chain constant region CH1; (b) a Fd fragment consisting of VH and CH1 domains; (c) an Fv fragment consisting of VL and VH domains of a single antibody; (d) a dAb fragment consisting of a VH domain (Ward ES et al., Nature 341:544-546 (1989)); (e) an isolated CDR region; (f) a F(ab')2 fragment, which is a bivalent fragment including two linked Fab fragments; (g) a single-chain Fv molecule (scFv) in which the VH domain and the VL domain are linked to each other by a peptide linker to form an antigen-binding domain; (h) a bispecific single-chain Fv dimer (PCT/US92/09965); and (i) a diabody (WO94/13804), which is a multivalent or multispecific fragment produced by gene fusion.

The antibody or the immunologically active fragment thereof of the present invention may be selected from the group consisting of animal-derived antibodies, chimeric antibodies, humanized antibodies, human antibodies, and immunologically active fragments thereof. The antibody may be produced recombinantly or synthetically.

Animal-derived antibodies produced by immunizing an animal to be immunized with a desired antigen may generally cause immune rejection when administered to humans for therapeutic purposes, and chimeric antibodies have been developed to suppress such immune rejection. The chimeric antibody is obtained by substituting a constant region of an animal-derived antibody, which causes an anti-isotype response, with a constant region of a human antibody using a genetic engineering method. Compared to animal-derived antibodies, the chimeric antibody is improved significantly in the anti-isotype response, but includes the side effects on potential anti-idiotypic responses because animal-derived amino acids are still present in a variable region. The humanized antibody is developed to improve these side effects. The humanized antibody is produced by grafting complementarity determining regions (CDRs), which play an important role in antigen binding in the variable regions of the chimeric antibody, to a human antibody framework.

In the CDR grafting technology for producing the humanized antibody, it is most important to select an optimized human antibody that may best accept the CDR region of the animal-derived antibody, and to this end, applications of an antibody database, analysis of a crystal structure, molecular modeling technology, etc. are used. However, even if the CDR region of an animal-derived antibody is grafted into an optimized human antibody framework, in some cases, there are amino acids that affect antigen binding while being located in the framework of the animal-derived antibody. As a result, since there are many cases in which antigen-binding affinity is not preserved, it is required to apply additional antibody engineering techniques to restore the antigen-binding affinity.

The antibody or the immunologically active fragment thereof may be isolated from a living body (not present in a living body) or may non-naturally occur, for example, synthetically or recombinantly produced.

In the present invention, the "antibody" refers to a substance produced by stimulation of an antigen in the immune system, and the type thereof is not particularly limited, and may be obtained naturally or non-naturally (e.g., synthetically or recombinantly). The Antibody is advantageous for mass expression and production because of being very stable in vivo as well as in vitro and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and subclasses include gamma 1 (γ1), gamma 2 (γ2), gamma 3 (γ3), gamma 4 (γ4), alpha 1 (α1) and alpha 2 (α2). The light chain constant region has kappa (κ) and lambda (λ) types.

As used herein, the term "heavy chain" is interpreted as meaning including all of a full-length heavy chain including a variable region domain V_{H} including an amino acid sequence having a sufficient variable region sequence to impart specificity to an antigen, three constant region domains C_{H1}, C_{H2} and C_{H3} and hinges, and fragments thereof. In addition, the term "light chain" is interpreted as meaning including all of a full-length light chain including a variable region domain V_{L} including an amino acid sequence having a sufficient variable region sequence to impart specificity to an antigen and a constant region domain C_{L}, and fragments thereof.

In the present invention, the term "variable region or variable domain" refers to a part of an antibody molecule that exhibits many variations on the sequence while performing a function that specifically binds to an antigen, and in the variable region, there are the complementarity determining regions CDR1, CDR2 and CDR3. A framework region (FR) portion exists between the CDRs to support a CDR ring. The "complementarity determining region" is a ring-shaped region involved in antigen recognition, and the specificity of the antibody for the antigen is determined as the sequence of this region changes.

The term "single chain fragment variable (scFv)" used herein refers to a single-chain antibody formed by expressing only a variable region of the antibody through genetic recombination, and means a single-chain form in which the VH and VL regions of the antibody are linked to each other by a short peptide chain. The term "scFv" is intended to include a scFv fragment including an antigen-binding fragment, unless otherwise specified or otherwise understood from the context. This is obvious to those skilled in the art.

In the present invention, the term "complementarity determining region (CDR)" refers to an amino acid sequence of a hypervariable region of the heavy and light chains of immunoglobulin. The heavy and light chains may include three CDRs (CDRH1, CDRH2, CDRH3 and CDRL1, CDRL2, CDRL3), respectively. The CDRs may provide key contact residues for the antibody binding to an antigens or epitope.

In the present invention, the term "specifically binding" or "specifically recognizing" has the same meaning as commonly known to those skilled in the art, and means that an antigen and an antibody specifically interact with each other to cause an immunological response.

As used herein, the term "antigen-binding fragment" refers to a fragment of the entire structure of immunoglobulin, and refers to a portion of a polypeptide including a portion capable of binding with the antigen. For example, the antigen-binding fragment may be scFv, (scFv)2, scFv-Fc, Fab, Fab' or F(ab')2, but is not limited thereto. The Fab of the antigen binding fragments has a structure having variable regions of the light and heavy chains, a constant region of the light chain, and a first constant region (C_{H1}) of the heavy chain, and has one antigen binding domain. The Fab' is different from Fab in that there is a hinge-region containing one or more cysteine residues at a C-terminal of the heavy chain C_{H1} domain. The F(ab')2 antibody is produced when the cysteine residues in the hinge region of Fab' form disulfide bonds. Fv is a minimal antibody fragment having only a heavy chain variable region and a light chain variable region, and a recombination technique for generating the Fv fragment is widely known in the art. The two-chain Fv has the heavy chain variable region and the light chain variable region linked via a noncovalent bond, and the single-chain Fv may generally form a dimer-like structure, such as the two-chain Fv because the variable region of the heavy chain and the variable region of the single chain are covalently linked via a peptide linker or directly linked at a C-terminal. The linker may be a peptide linker consisting of 1 to 100 or 2 to 50 random amino acids, and appropriate sequences are known in the art. The antigen binding fragments may be obtained using protease (for example, the whole antibody is restriction-cleaved with papain to obtain Fab, and cleaved with pepsin to obtain an F(ab')2 fragment), or may be produced through genetic recombination technology.

In the present invention, the term "hinge region" refers to a region included in the heavy chain of the antibody, and means a region which is present between the C_{H1} and C_{H2} regions and functions to provide flexibility of the antigen binding domain in the antibody. For example, the hinge may be derived from a human antibody, specifically, derived from IgA, IgE, or IgG, such as IgG1, IgG2, IgG3, or IgG4.

In one aspect, the present invention related to a bispecific or multispecific antibody including the antibody specific for the Fc alpha receptor or the immunologically active fragment thereof of the present invention, and a moiety that binds to a target antigen other than the Fc alpha receptor.

In an exemplary embodiment, the moiety that binds to the target antigen may include an antibody or an immunologically active fragment thereof, and the immunologically active fragment may be any one selected from the group consisting of Fab, Fd, Fab', dAb, F(ab'), F(ab')₂, scFv, Fv, a single chain antibody, a Fv dimer, a complementarity determining region fragment, a humanized antibody, a chimeric antibody, and a diabody, and more preferably scFv or Fab.

In an exemplary embodiment, the target antigen may be at least one selected from the group consisting of 17-1A antigen, GD3 ganglioside R24, EGFRvIII, PSMA, PSCA, HLA-DR, EpCAM, MUC1 core protein, aberrant glycosylation MUC1, fibronectin heteroform containing ED-B domain, HER2/neu, carcinoembryonic antigen (CEA), gastrin-releasing peptide (GRP) receptor antigen, mucine antigen, epidermal growth factor receptor (EGF-R), HER3, HER4, MAGE antigen, SART antigen, MUC1 antigen, c-erb-2 antigen, TAG 72, carbonic anhydrase IX, alpha-fetoprotein, A3, an antigen specific to an A33 antibody, Ba 733, BrE3-antigen, CA125, CDl, CD1a, CD3, CD5, CDl5, CD16, CD19, CD20, CD21, CD22, CD23, CD25, CD30, CD33, CD38, CD40, CD45, CD52, CD74, CD79a, CD80, CD138, colon-specific antigen-p (CSAp), CSAp, EGP-1, EGP-2, Ep-CAM, FIt-1, Flt-3, folate receptor, HLA-DR, human chorionic gonadotropin (HCG) and its subunits, hypoxia inducible factor (HIF-I), Ia, IL-2, IL-6, IL-8, insulin growth factor-1 (IGF-1), KC4-antigen, KS-1-antigen, KSl-4, Le-Y, macrophage inhibitory factor (MIF), MAGE, MUCl, MUC2, MUC3, MUC4, NCA66, NCA95, NCA90, antigen specific for PAM-4 antibody, placental growth factor, p53, prostatic acid phosphatase, PSA, RS5, SlOO, TAC, tenascin, TRAIL receptors, Tn antigen, Thomson-Friedenreich antigens, tumor necrosis antigens, VEGF, ED-B fibronectin, angiogenesis marker, oncogene marker or oncogene product. The oncogene may be an apoptosis-related gene, a transcription factor gene, a metastasis-related gene, an angiogenesis-related gene, or a tyrosine-kinase gene.

In an exemplary embodiment, the apoptosis-related gene may be ABL1, AKT1, AKT2, BARD1, BAX, BCL11B, BCL2, BCL2A1, BCL2L1, BCL2L12, BCL3, BCL6, BIRC2, BIRC3, BIRC5, BRAF, CARD11, CAV1, CBL, CDC25A, CDKN1A, CFLAR, CNR2, CTNNB1, CUL4A, DAXX, DDIT3, E2F1, E2F3, E2F5, ESPL1, FOXO1, HDAC1, HSPA5, IGF1R, IGF2, JUN, JUNB, JUND, MALT1, MAP3K7, MCL1, MDM2, MDM4, MYB, MYC, NFKB2, NPM1, NTRK1, PAK1, PAX3, PML, PRKCA, PRKCE, PTK2B, RAF1, RHOA, TGFB1, TNFRSF1B, TP73, TRAF6, YWHAG, YWHAQ or YWHAZ. The transcription factor gene may be AR, ARID3A, ASCL1, ATF1, ATF3, BCL11A, BCL11B, BCL3, BCL6, CDC5L, CDX2, CREB1, CUX1, DDIT3, DLX5, E2F1, E2F3, E2F5, ELF4, ELK1, ELK3, EN2, ERG, ETS1, ETS2, ETV1, ETV3, ETV4, ETV6, FEV, FEZF1, FLI1, FOS, FOSL1, FOXA1, FOXG1, FOXM1, FOXO1, FOXP1, FOXQ1, GATA1, GATA6, GFI1, GFI1B, GLI1, GLI2, GLI3, HES6, HHEX, HLF, HMGA1, HMGA2, HOXA1, HOXA9, HOXD13, HOXD9, ID1, ID2, IKZF1, IRF2, IRF4, JUN, JUNB, JUND, KAT6A, KDM2A, KDM5B, KLF2, KLF4, KLF5, KLF6, KLF8, KMT2A, LEF1, LHX1, LMX1B, MAF, MAFA, MAFB, MBD1, MECOM, MEF2C, MEIS1, MITF, MYB, MYC, MYCL, MYCN, NANOG, NCOA3, NFIB, NFKB2, NKX2-1, OTX2, PATZ1, PAX2, PAX3, PAX4, PAX8, PBX1, PBX2, PITX2, PLAG1, PLAGL2, PPARG, PPP1R13L, PRDM10, PRDM13, PRDM14, PRDM15, PRDM16, PRDM6, PRDM8, PRDM9, RARA, REL, RERE, RUNX1, RUNX3, SALL4, SATB1, SFPQ, SIX1, SNAI1, SOX2, SOX4, SPI1, SREBF1, STAT3, TAF1, TAL1, TAL2, TBX2, TBX3, TCF3, TFCP2, TFE3, THRA, TLX1, TP63, TP73, TWIST1, WT1, YBX1, YY1, ZBTB16, ZBTB7A, ZIC2, ZNF217 or ZNF268. The metastasis-related gene may be AKT1, AKT2, AR, CBL, CDH1, CRK, CSF1, CTNNB1, CTTN, CXCR4, EGFR, FGFR1, FLT3, FYN, GLI1, ILK, ITGA3, JAK2, MET, PDGFRB, PLXNB1, PRKCI, PTCH1, PTPN11, RAC1, RHOA, RHOC, ROCK1, SMO, SNAI1, SRC, TCF3 or WT1. The angiogenesis-related gene may be BRAF, CAV1, CTGF, EGFR, ERBB2, ETS1, FGF4, FGF6, FGFR1, FGFR3, FGFR4, ID1, NRAS, PDGFB, PDGFRA, PDGFRB or SPARC. The tyrosine-kinase gene may be ABL1, ABL2, ALK, AXL, BLK, EGFR, EPHA2, ERBB2, ERBB3, ERBB4, FES, FGFR1, FGFR2, FGFR3, FGFR4, FGR, FLT3, FYN, ITK, JAK1, JAK2, KIT, LCK, MERTK, MET, MST1R, NTRK1, NTRK3, PDGFRA, PDGFRB, PTK2B, PTK7, RET, ROS1, SRC, SYK, TEC or YES 1.

In an exemplary embodiment, the oncogene may be SEPTIN9, ACOD1, ACTN4, ADAM28, ADAM9, ADGRF1, ADRBK2, AFF1, AFF3, AGAP2, AGFG1, AGRN, AHCYL1, AHI1, AIMP2, AKAP13, AKAP9, AKIRIN2, AKTIP, ALDH1A1, ALL1, ANIB1, ANP32C, ANP32D, AQP1, ARAF, ARHGEF1, ARHGEF2, ARHGEF5, ASPSCR1, AURKA, BAALC, BAIAP2L1, BANP, BCAR4, BCKDHB, BCL9, BCL9L, BCR, BMI1, BMP7, BOC, BRD4, BRF2, CABIN1, CAMK1D, CAPG, CBFB, CBLB, CBLL1, CBX7, CBX8, CCDC28A, CCDC6, CCNB1, CCNB2, CCND1, CCNE1, CCNL1, CD24, CDC25C, CDC6, CDH17, CDK1, CDK14, CDK4, CDK5R2, CDK6, CDK8, CDKN1B, CDKN3, CDON, CEACAM6, CENPW, CHD1L, CHIC1, CHL1, CKS1B, CMC4, CNTN2, COPS3, COPS5, CRKL, CRLF2, CROT, CRTC1, CRYAB, CSF1R, CSF3, CSF3R, CSNK2A1, CSNK2A2, CT45A1, CTBP2, CTNND2, CTSZ, CUL7, CXCL1, CXCL2, CXCL3, CYGB, CYP24A1, DCD, DCUN1D1DDB2, DDHD2, DDX6, DEK, DIS3, DNPH1, DPPA2, DPPA4, DSG3, DUSP12, DUSP26, ECHS1, ECT2, EEF1A1, EEF1A2, EEF1D, EIF3E, EIF3I, EIF4E, EIF5A2, ELAVL1, ELL, EML4, EMSY, ENTPD5, EPCAM, EPS8, ERAS, ERGIC1, ERVW-1, EVI2A, EVI5, EWSR1, EZH2, FAM189B, FAM72A, FAM83D, FASN, FDPS, FGF10, FGF3, FGF5, FGF8, FR1OP, FHL2, FIP1L1, FNDC3B, FRAT1, FUBP1, FUS, FZD2, GAB2, GAEC1, GALNT10, GALR2, GLO1, GMNN, GNA12, GNA13, GNAI2, GNAQ, GNAS, GOLPH3, GOPC, GPAT4, GPM6A, GPM6B, GPR132, GREM1, GRM1, GSK3A, GSM1, H19, HAS1, HAX1, HDGFRP2, HMGN5, HNRNPA1, HOTAIR, HOTTIP, HOXA-AS2, HRAS, HSPA1A, HSPA4, HSPB1, HULC, IDH1, IFNG, IGF2BP1, IKBKE, IL7R, INPPL1, INTS1, INTS2, INTS3, INTS4, INTS5, INTS7, INTS8, IRS2, IST1, JUP, KDM4C, KIAA0101, KIAA1524, KIF14, KRAS, KSR2, LAMTOR5, LAPTM4B, LCN2, LDHB, LETMD1, LIN28A, LIN28B, LMO1, LMO2, LMO3, LMO4, LSM1, LUADT1, MACC1, MACROD1, MAGEA11, MALAT1, MAML2, MAP3K8, MAPRE1, MAS1, MCC, MCF2, MCF2L, MCTS1, MEFV, MFHAS1, MFNG, MIEN1, MINA, MKL2, MLANA, MLLT1, MLLT11, MLLT3, MLLT4, MMP12, MMS22L, MN1, MNAT1, MOS, MPL, MPST, MRAS, MRE11A, MSI1, MTCP1, MTDH, MTOR, MUC1, MUC4, MUM1, MYD88, NAAA, NANOGP8, NBPF12, NCOA4, NEAT1, NECTIN4, NEDD4, NEDD9, NET1, NINL, NME1, NOTCH1, NOTCH4, NOV, NSD1, NUAK2, NUP214, NUP98, NUTM1, OLR1, PA2G4, PADI2, PAK7, PARK7, PARM1, PBK, PCAT1, PCAT5, PDGFA, PDZK1IP1, PELP1, PFN1P3, PIGU, PIK3CA, PIK3R1, PIM1, PIM2, PIM3, PIR, PIWIL1, PLAC8, PLK1, PPM1D, PPP1R10, PPP1R14A, PPP2R1A, PRAME, PRDM12, PRMT5, PSIP1, PSMD10, PTCH2, PTMA, PTP4A1, PTP4A2, PTP4A3, PTTG1, PTTG1IP, PTTG2, PVT1, RAB11A, RAB18, RAB22A, RAB23, RAB8A, RALGDS, RAP1A, RASSF1, RBM14, RBM15, RBM3, RBMY1A1, RFC3, RGL4, RGR, RHO, RING1, RINT1, RIT1, RNF43, RPL23, RRAS, RRAS2, RSF1, RUNX1T1, S100A4, S100A7, S100A8, SAG, SART3, SBSN, SEA, SEC62, SERTAD1, SERTAD2, SERTAD3, SET, SETBP1, SETDB1, SGK1, SIRT1, SIRT6, SKI, SKIL, SKP2, SLC12A5, SLC3A2, SMR3B, SMURF1, SNCG, SNORA59A, SNORA80E, SPAG9, SPATA4, SPRY2, SQSTM1, SRSF1, SRSF2, SRSF3, SRSF6, SS18, SSX1, SSX2, SSX2B, STIL, STMN1, STRA6, STYK1, SUZ12, SWAP70, SYT1, TAC1, TACSTD2, TAF15, TALDO1, TAZ, TBC1D1, TBC1D15, TBC1D3, TBC1D3C, TBC1D7, TCL1A, TCL1B, TCL6, TCP1, TFG, TGM3, TINCR, TKTL1, TLE1, TMEM140, TMPOP2, TMPRSS2, TNS4, TPD52, TPR, TRE17, TREH, TRIB1, TRIB2, TRIM28, TRIM32, TRIM8, TRIO, TRIP6, TSPAN1, TSPY1, TXN, TYMS, TYRP1, UBE2C, UBE3C, UCA1, UCHL1, UHRF1, URI1, USP22, USP4, USP6, VAV1, VAV2, VAV3, VIM, WAPL, WHSC1, WHSC1L1, WISP1, WNT1, WNT10A, WNT10B, WNT2, WNT3, WNT5A, WWTR1, XCL1, XIAP, YAP1, YEATS4, YY1AP1, ZEB1-AS1, ZFAND4, ZFAS1, ZMYM2, ZNF703 or ZNHIT6.

In an exemplary embodiment, the target antigen may be a cell surface antigen or an autoantigen, and the cell surface antigen may be at least one selected from the group consisting of CEA, ED-B fibronectin, CD20, CD22, CDl9, EGFR, IGFlR, VEFGRl/Flt-1, VEGFR2/KDR, VEGRF3/Flt-4, HER2/neu, CD30, CD33, CD3, CD16, CD64, CD89, CD2, adenovirus fiber knob, PfMSP-1, HN/NDV, EpCAM/17-lA, hTR, IL-2R/Tac, CAl9-9, MUCl, HLA class II, GD2, G250, TAG-72, PSMA, CEACAM6, HMWMAA, CD40, Ml3 enveloped protein and GPIIb/IIIa.

In one aspect, the present invention relates to an isolated nucleic acid molecule encoding the antibody or the immunologically active fragment thereof of the present invention or the bispecific or multispecific antibody of the present invention, a vector including the isolated nucleic acid molecule, and a host cell transformed with the vector.

The nucleic acid molecule of the present invention may be isolated or recombinant, and includes not only DNA and RNA in single-stranded and double-stranded form, but also corresponding complementary sequences. The isolated nucleic acid is a nucleic acid that has been isolated from surrounding genetic sequences present in the genome of a subject from which the nucleic acid was isolated, in the case of a nucleic acid isolated from a naturally occurring source. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when placed in a functional relationship with another nucleic acid sequence. For example, DNA of a pre-sequence or secretory leader is expressed as a preprotein in the form before the polypeptide is secreted to be operably linked to DNA of the polypeptide, and a promoter or enhancer is operably linked to a coding sequence when affecting the transcription of the polypeptide sequence, or a ribosome binding domain is operably linked to a coding sequence when disposed to promote the translation. In general, the operably linked means that DNA sequences to be linked are located contiguously, and that the secretory leader exists contiguously in the same reading frame. However, the enhancer needs not to be contiguously located. The linkage is achieved by ligation at a convenient restriction enzyme site. When there is no site, synthetic oligonucleotide adapters or linkers are used according to conventional methods.

In the isolated nucleic acid molecule encoding the antibody or the immunologically active fragment thereof of the present invention or the bispecific or multispecific antibody of the present invention, due to the degeneracy of codons or in consideration of codons preferred in an organism in which the antibody is to be expressed, it will be well understood to those skilled in the art that various modifications may be made in a coding region within a range without changing the amino acid sequence of the antibody to be expressed from the coding region, various modifications or changes may be made within a range without affecting the expression of the gene even in parts other than the coding region, and such modified genes are also included within the scope of the present invention. That is, as long as the nucleic acid molecule of the present invention encodes a protein having equivalent activity thereto, one or more nucleobases may be mutated by substitution, deletion, insertion, or a combination thereof, which are also included in the scope of the present invention. The sequence of such a nucleic acid molecule may be single- or double-stranded, and may be a DNA molecule or an RNA (mRNA) molecule.

According to the present invention, the isolated nucleic acid molecule encoding the antibody or the immunologically active fragment thereof of the present invention or the bispecific or multispecific antibody of the present invention may be inserted into an expression vector for protein expression. The expression vector generally includes a protein operably linked, i.e., functionally related with a control or regulatory sequence, a selectable marker, an optional fusion partner, and/or an additional element. In appropriate conditions, the antibody or the immunologically active fragment thereof of the present invention or the bispecific or multispecific antibody of the present invention may be produced by a method of inducing the protein expression by culturing a host cell transformed with a nucleic acid, preferably an expression vector containing an isolated nucleic acid molecule encoding the antibody or the immunologically active fragment thereof of the present invention or the bispecific or multispecific antibody of the present invention. A variety of suitable host cells including mammalian cells, bacteria, insect cells, and yeast may be used, but are not limited thereto. Methods for introducing exogenous nucleic acids into host cells are known in the art and will vary depending on a host cell to be used. Preferably, it is possible to produce E. coli, which has high industrial value due to low production cost, as a host cell.

The vector of the present invention may include a plasmid vector, a cosmid vector, a bacteriophage vector, a viral vector, etc., but is not limited thereto. The suitable vector includes a signal sequence or a leader sequence for membrane targeting or secretion in addition to expression regulatory elements such as a promoter, an operator, an initiation codon, a termination codon, a polyadenylation signal, and an enhancer and may be variously produced according to a purpose. The promoter of the vector may be constitutive or inductive. The signal sequence may use a PhoA signal sequence, an OmpA signal sequence, etc. in the case of Escherichia sp. bacteria as a host, an α-amylase signal sequence, a subtilisin signal sequence, etc. in the case of Bacillus sp. bacteria as a host, an MFα signal sequence, a SUC2 signal sequence, etc. in the case of yeast as a host, and an insulin signal sequence, an α-interferon signal sequence, an antibody molecule signal sequence, etc. in the case of an animal cell as a host, but is not limited thereto. Further, the vector may include a selective marker for selecting a host cell including a vector and a replicable expression vector includes a replication origin.

As used herein, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (e.g., bacteriophage), but is not limited thereto. Those skilled in the art may construct vectors by standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994 etc.).

In an exemplary embodiment, when constructing the vector, expression regulatory sequences such as a promoter, a terminator, and an enhancer, sequences for membrane targeting or secretion, etc. are appropriately selected according to a type of host cell and may be variously combined depending on a purpose.

In the present invention, the term "expression vector" refers to a vector including a nucleic acid sequence encoding at least a portion of a gene product to be transcribed. In some cases, the RNA molecule is then translated into a protein, a polypeptide, or a peptide. The expression vector may include various regulatory sequences. In addition to regulatory sequences that regulate transcription and translation, vectors and expression vectors may also include nucleic acid sequences that serve other functions.

In the present invention, the term "host cell" includes a eukaryote and a prokaryote, and refers to any transformable organism capable of replicating the vector or expressing a gene encoded by the vector. The host cell may be transfected or transformed by the vector, which means a process in which an exogenous nucleic acid molecule is delivered or introduced into the host cell.

In an exemplary embodiment, the host cell may be a bacterial or animal cell, the animal cell line may be a CHO cell, a HEK cell or a NSO cell, and the bacteria may be Escherichia coli.

In one aspect, the present invention relates to a pharmaceutical composition for preventing or treating a disease or disorder selected from the group consisting of cancer, autoimmune disease, neurodegerative disease, Alzheimer's disease, metabolic disease, cardiovascular disease, atherosclerosis, organ transplant rejection, and diseases or symptoms caused by fungi, viruses, bacteria or parasites, including an antibody or an immunologically active fragment thereof of the present invention, and a bispecific or multispecific antibody including a region that binds to a target antigen other than an Fc alpha receptor as an active ingredient.

In an exemplary embodiment, the cancer may be any one selected from the group consisting of brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas.

In the present invention, the term "prevention" refers to all actions that inhibit or delay the occurrence, spread, and recurrence of the disease or disorder by administration of the composition according to the present invention.

As used herein, the term "treatment" means all actions that improve or beneficially change the symptoms of the disease or disorder and its complications by administration of the composition according to the present invention. Those skilled in the art to which the present invention pertains will be able to determine the degree of improvement, enhancement and treatment by knowing the exact criteria of a disease for which the composition of the present invention is effective by referring to data presented by the Korean Academy of Medical Sciences, etc.

In the present invention, the term "therapeutically effective dose" used in combination with the active ingredients means an amount effective to prevent or treat the disease or disorder, and the therapeutically effective dose of the composition of the present invention may vary depending on several factors, such as a method of administration, a target site, the condition of a patient. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective dose. As used herein, the term "pharmaceutically effective dose" refers to an amount enough to treat diseases at a reasonable benefit/risk ratio applicable to medical treatment and enough to not cause side effects. The effective dose level may be determined according to factors including a health condition of a patient, a type of disease or disorder, the severity of a disease or disorder, drug activity, sensitivity to drug, an administration method, an administration time, an administration route and excretion rate, a treatment period, and drugs used in combination or concurrently, and other factors well-known in medical fields. The composition of the present invention may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may include a carrier, diluent, excipient, or a combination of two or more thereof, which are commonly used in biological agents. As used herein, the term "pharmaceutically acceptable" refers to exhibiting non-toxic properties to cells or humans exposed to the composition. The carrier is not particularly limited as long as the carrier is suitable for in vivo delivery of the composition, and may be used with, for example, compounds described in Merck Index, 13th ed., Merck & Co. Inc., saline, sterile water, a Ringer's solution, buffered saline, a dextrose solution, a maltodextrin solution, glycerol, ethanol, and mixtures of one or more of these components, and if necessary, other conventional additives such as an antioxidant, a buffer, and a bacteriostat may be added. In addition, the pharmaceutical composition of the present invention may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition of the present invention may be preferably prepared according to each disease or ingredient using a suitable method in the art or a method disclosed in Remington's Pharmaceutical Science (Mack Publishing Company, Easton PA, 18th, 1990).

In an exemplary embodiment, the pharmaceutical composition may be one or more formulations selected from the group including oral formulations, external formulations, suppositories, sterile injection solutions and sprays, and oral formulations or injectable formulations are more preferred.

As used herein, the term "administration" means providing a predetermined substance to a subject or patient by any suitable method, and the pharmaceutical composition may be administered parenterally (applied as the injectable formulations, e.g., intravenously, subcutaneously, intraperitoneally or topically) or orally according to a desired method, and the dose range may vary depending on the body weight, age, sex, and health condition of a patient, a diet, an administration time, an administration method, an excretion rate, and the severity of a disease. Liquid formulations for oral administration of the composition of the present invention correspond to suspensions, internal solutions, emulsions, syrups, etc., and may include various excipients, such as wetting agents, sweeteners, fragrances, preservatives, and the like in addition to water and liquid paraffin, which are commonly used simple diluents. Formulations for parenteral administration include sterilized aqueous solutions, non-aqueous solvents, suspensions, emulsions, lyophilized agents, suppositories, and the like. The pharmaceutical composition of the present invention may also be administered by any device capable of transferring an active substance to a target cell. Preferred methods of administration and formulations are intravenous injections, subcutaneous injections, intradermal injections, intramuscular injections, or drop injections. The injections may be prepared by using aqueous solvents such as a physiological saline solution and a ringer solution, and non-aqueous solvents such as vegetable oils, higher fatty acid esters (e.g., ethyl oleate), and alcohols (e.g., ethanol, benzyl alcohol, propylene glycol, glycerin, etc.). The injections may include pharmaceutical carriers, such as a stabilizer for the prevention of degeneration (e.g., ascorbic acid, sodium hydrogen sulfite, sodium pyrosulfite, BHA, tocopherol, EDTA, etc.), an emulsifier, a buffer for pH control, and a preservative to inhibit microbial growth (e.g., phenyl mercury nitrate, thimerosal, benzalkonium chloride, phenol, cresol, benzyl alcohol, etc.).

As used herein, the term "subject" refers to any animal including monkeys, cows, horses, sheep, pigs, chickens, turkeys, quails, cats, dogs, mice, bats, camel, rat, rabbit or guinea pig including humans who have developed or may develop the disease or disorder, and the "specimen" may be droplet, sputum, whole blood, plasma, serum, urine or saliva isolated therefrom.

The pharmaceutical composition of the present invention may further include a pharmaceutically acceptable additive. At this time, the pharmaceutically acceptable additive may use starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present invention is preferably included in an amount of 0.1 part by weight to 90 parts by weight based on the composition, but is not limited thereto.

In an aspect, the present invention relates to a method for providing information on diagnosis of a disease or disorder selected from the group consisting of cancer, autoimmune disease, neurodegerative disease, Alzheimer's disease, metabolic disease, cardiovascular disease, atherosclerosis, organ transplant rejection, and diseases or symptoms caused by fungi, viruses, bacteria or parasites, including the steps of forming an antigen-antibody complex by contacting a sample isolated from a specimen with the bispecific or multispecific antibody of the present invention; and detecting the formation of the complex.

In one aspect, the present invention relates to a method for producing an antibody specific for the Fc alpha receptor or an immunologically active fragment thereof including the steps of culturing a host cell transformed with a vector including an isolated nucleic acid molecule encoding the antibody or the immunologically active fragment thereof of the present invention; and recovering the antibody or the immunologically active fragment thereof from the host cell culture.

In one aspect, the present invention relates to a method for producing a bispecific or multispecific antibody binding to an Fc alpha receptor and a target antigen other than the Fc alpha receptor including the steps of culturing a host cell transformed with a vector including an isolated nucleic acid molecule encoding the bispecific or multispecific antibody of the present invention; and recovering the antibody or the immunologically active fragment thereof from the host cell culture.

In one aspect, the present invention relates to a use of the antibody specific for the Fc alpha receptor or the immunologically active fragment thereof of the present invention for use in the preparation of an antibody therapeutic agent.

In one aspect, the present invention relates to a use of the bispecific or multispecific antibody of the present invention for prevention or treatment of a disease or disorder selected from the group consisting of cancer, autoimmune disease, neurodegerative disease, Alzheimer's disease, metabolic disease, cardiovascular disease, atherosclerosis, organ transplant rejection, and diseases or symptoms caused by fungi, viruses, bacteria or parasites.

In one aspect, the present invention relates to a method for treating cancer including administering the bispecific or multispecific antibody of the present invention in a pharmaceutically effective dose to a subject suffering from cancer.

### [Modes of the Invention]

Hereinafter, the present invention will be described in more detail with reference to the following Examples. However, the following Examples are only intended to embody the contents of the present invention, and the present invention is not limited thereto.

### Example 1. Preparation of antigen using Fc alpha receptor

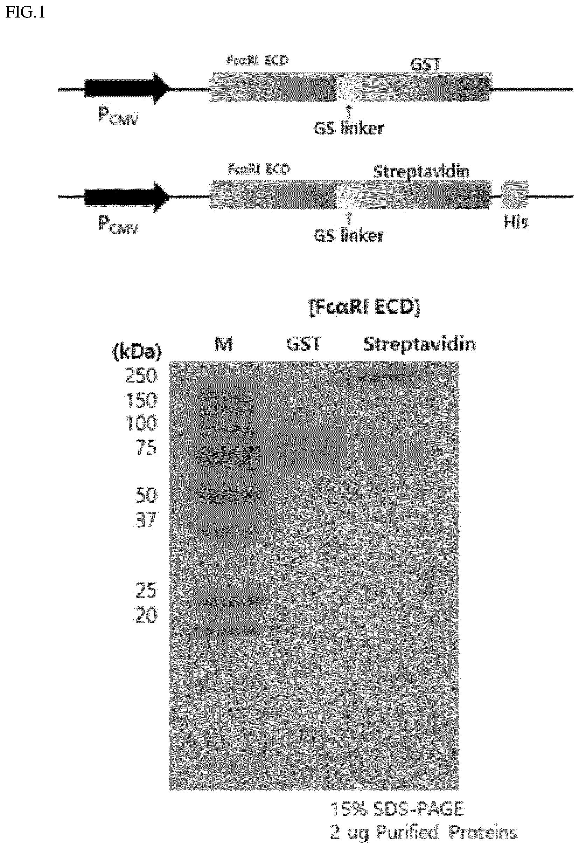

In order to discover IgG having an effector function by binding to an Fc alpha receptor, an extra cellular domain (ECD) of the Fc alpha receptor was prepared as an antigen. In addition, in order to produce an antigen suitable for screening an antibody using a flow cytometer, a vector for animal cell expression was constructed to produce antigens in a dimeric form fused with a Glutathione-S-transferase (GST) tag and a tetramer form fused with a streptavidin tag (FIG. 1). Then, the vector was transfected to Expi293F cells cultured for one day at a density of 2 × 10⁶ cells/ml using PEI (Polyscience, 23966). Thereafter, the transfected cells were incubated for 7 days under conditions of 37°C, 125 rpm, and 8 % CO₂ in a shaking incubator, and then centrifuged to collect only a supernatant. The supernatant was equilibrated with 25x PBS and filtered through a 0.2 µm filter (Merck Millipore) using a bottle top filter. The filtered culture solution was added with 1 ml of GSH resin and Ni-NTA resin and stirred at 4°C for 16 hours, and then the resin was recovered through the column, washed with 5 ml PBS and eluted with 4 ml of 50 mM Tris-HCl & 10 mM reduced glutathione (SIGMA) at pH 8.0 and 250 mM imidazole. The buffer was replaced with PBS using centrifugal filter units 3K (Merck Millipore), and then the size and purity of two purified antigen proteins GST-ECD and streptavidin-ECD were analyzed by SDS-PAGE (FIG. 1).

### Example 2. Human antibody screening using high-speed search system.

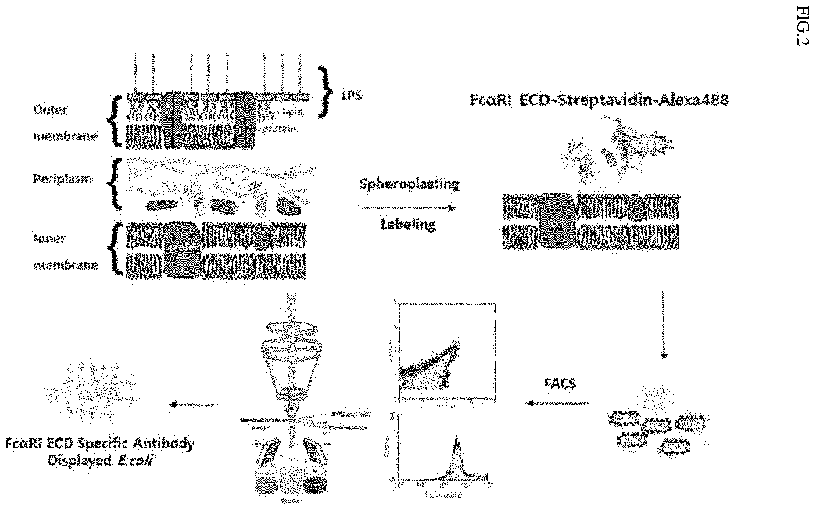

For use in screening using a flow cytometer, among the antigen proteins prepared in Example, the antigen proteins fused with streptavidin were labeled with Alexa488 fluorescent molecules. Thereafter, human antibodies binding to the ECD region of the Fc alpha receptor were screened using a bacteria-displayed human scFv antibody library constructed by the present inventors. Specifically, 1 ml of the library cells were incubated in 25 ml of a TB medium containing 2% glucose and 40 µg/ml of chloramphenicol at 37°C and 250 rpm for 4 hours, and the incubated cells were incubated in 100 ml of a TB medium containing 40 µg/ml of chloramphenicol at a ratio of 1 : 100 to be OD₆₀₀ = 0.5 and cooled at 25°C and 250 rpm for 20 minutes, and then added with 1 mM IPTG and induced at 25°C and 250 rpm for 5 hours to harvest protein-overexpressing E. coli based on OD₆₀₀ = 8. In order to make the cells into spheroplasts suitable for screening using the flow cytometer, the cells were resuspended using 1 ml of 10 Mm Tris-HCl (pH 8.0) and washed twice to remove the remaining medium, and then rotated in 1 ml of a STE [0.5 M sucrose, 10 Mm Tris-HCl, and 10 Mm EDTA (pH 8.0)] solution at 37°C for 30 minutes to remove the cell outer membranes by an osmotic shock. Thereafter, the cells were rotated in a mixed solution of 1 ml of Solution A and 20 µl of 50 mg/ml lysozyme solution at 37°C for 15 minutes to remove a peptidoglycan layer, and washed with 1 ml of PBS, and then 300 µl of the cells were added with 700 µl of PBS and 200 nM (based on monomer) of an Fc alpha receptor-ECD-streptavidin-Alexa488 probe and rotated at room temperature for 1 hour to label spheroplasts with fluorescent probes. Thereafter, clones showing high fluorescence due to increased binding affinity of antigen were recovered using the flow cytometer, and in the recovered clones, processes of amplifying scFv genes by PCR, culture, expression induction, spheroplasting to remove a cell outer membrane and a peptidoglycan layer, and selecting spheroplasts showing high affinity in the Fc alpha receptor-ECD region through selective gating of antigen labeling and flow cytometry were repeated. After the selection process using the flow cytometer and the re-selection process to increase the selection purity, an enrichment process, a process of securing the selected scFv variant gene through PCR amplification, a subcloning process, and a transformation process were performed, and the next round of selection was performed to enrich desired clones (FIG. 2). Through such a repeated search process at several rounds, scFv variant clones with excellent binding affinity for the Fc alpha receptor-ECD region were secured.

### Example 3. Identification of gene sequences of Fc alpha receptor-binding antibody variants

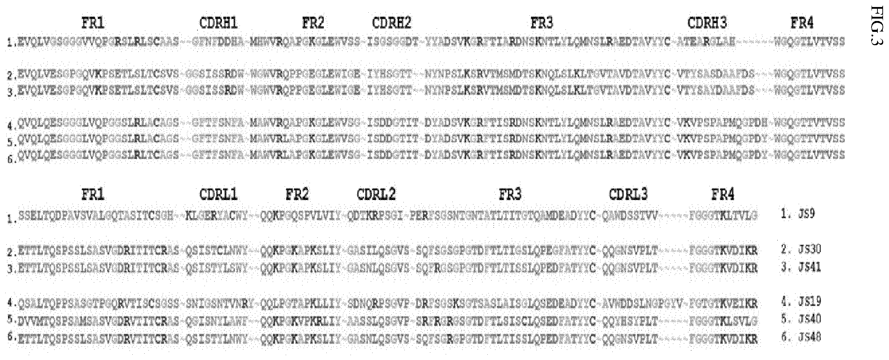

In order to identify the gene sequences of the scFv variant clones obtained in Example 2, DNA sequencing was analyzed by Sanger sequencing, and through the analysis, six scFv antibody variants JS9, JS19, JS30, JS40, JS41, and JS48 having antibody sequences were selected (FIG. 3). In addition, VH and VL amino acid sequences of the six scFv variants, and amino acid sequences of FR1, CDRH1, FR2, CDRH2, FR3, CDRH3 and FR4 of VH and amino acid sequences of FR1, CDRL1, FR2, CDRL2, FR3, CDRL3 and FR4 of VL of each variant were identified (Table 1).

**[Table 1]**

| Region | Variant name | | Sequence |
|---|---|---|---|
| VH | | | |
| CDRH1 | JS40-1, JS48-1, JS116, JS144, JS140, JS19, JS40, JS48 | GFTFSNFA | |
| | JS9 | GFNFDDHA | |
| | JS30, JS41 | GGSISSRDW | |
| CDRH2 | JS40-1, JS48-1, JS116, JS144, JS19, JS40, JS48 | ISDDGTIT | |
| | JS140 | ISDDGTTT | |
| | JS9 | ISGSGGDT | |
| | JS30, JS41 | IYHSGTT | |
| CDRH3 | JS116 | VRVPPPAPVQGPDY | |
| | JS144 | VRVPSPAPVQGPDY | |
| | JS140 | VRVPSPAPMQGPDY | |
| | JS40-1, JS48-1, JS40, JS48 | VKVPSPAPMQGPDY | |
| | JS9 | ATEARGLAH | |
| | JS30 | VTYSASDAAFDS | |
| | JS41 | VTYSAYDAAFDS | |
| | JS19 | VKVPSPAPMQGPDH | |

| VL | | | |
|---|---|---|---|
| CDRL1 | JS116, JS144, JS40-1, JS40 | QGISNYLAWF | |
| | JS140, JS48-1, JS48 | QSISTYLNWY | |
| | JS9 | KLGERYACWY | |
| | JS30 | QSISTCLNWY | |
| | JS41 | QSISTYLSWY | |
| | JS19 | SSNIGSNTVNRY | |
| CDRL2 | JS116, JS144 | AASSLQGGVP | |
| | JS140, JS48-1, JS41, JS48 | GASNLQSGVS | |
| | JS40-1, JS40 | AASSLQSGVP | |
| | JS9 | QDTKRPSGI | |
| | JS30 | GASILQSGVS | |
| | JS19 | SDNQRPSGVP | |
| CDRL3 | JS116, JS144, JS40-1, JS40 | QQYHSYPLT | |
| | JS140 | QQGNNVPLT | |
| | JS48-1, JS30, JS41, JS48 | QQGNSVPLT | |
| | JS9 | QAWDSSTVV | |
| | JS19 | AVWDDSLNGPGYV | |

| VH | | | |
|---|---|---|---|
| FR1 | JS116, JS144, JS48-1, JS40, JS19 | QVQLQESGGGLVQPGGSLRLACAGS | |
| | JS140, JS40-1, JS48 | QVQLQESGGGLVQPGGSLRLTCAGS | |
| | JS9 | EVQLVGSGGGVVQPGRSLRLSCAAS | |
| | JS30, JS41 | EVQLVESGPGQVKPSETLSLTCSVS | |
| FR2 | JS116, JS144, JS40-1, JS48-1, JS40, JS48 | MAWVRLAPGKGLEWVSG | |
| | JS140 | MAWVRLAPGEGLEWVSG | |
| | JS9 | MHWVRQAPGKGLEWVSS | |
| | JS30, JS41 | WGWVRQPPGEGLEWIGE | |
| | JS19 | MAW VRQAPGKGLEW VS G | |
| FR3 | JS116, JS144, JS140, | DYADSVKGRFTISRDNSKNTLYLQMNSLR | |
| | JS40-1, JS48-1, JS19, JS40, JS48 | AEDTAVYYC | |
| | JS9 | YYADSVKGRFTIARDNSKNTLYLQMNSLR AEDTAVYYC | |
| | JS30, JS41 | NYNPSLKSRVTMSMDTSKNQLSLKLTGVT AVDTAVYYC | |
| FR4 | JS116, JS144, JS48-1 | WGQGTTVTVSS | |
| | JS140, JS40-1, JS9, JS30, JS41, JS19, JS40, JS48 | WGQGTLVTVSS | |

| VL | | | |
|---|---|---|---|
| FR1 | JS116, JS144, JS48-1, JS40 | DVVMTQSPSAMSASVGDRVTITCRAS | |
| | JS140 | ETTLTQSPSSLSASVGDRVSITCRAS | |
| | JS40-1, JS48 | ETTLTQSPSSLSASVGDRVTITCRAS | |
| | JS9 | SSELTQDPAVSVALGQTASITCSGH | |
| | JS30, JS41 | ETTLTQSPSSLSASVGDRITITCRAS | |
| | JS19 | QSALTQPPSASGTPGQRVTISCSGS | |
| FR2 | JS116, JS144, JS48-1, JS40 | QQKPGKVPKRLIY | |
| | JS140, JS40-1, JS30, JS41, JS48 | QQKPGKAPKSLIY | |
| | JS9 | QQKPGQSPVLVIY | |
| | JS19 | QQLPGTAPKLLIY | |
| FR3 | JS116, JS144, JS48-1, JS40 | SRFRGRGSGTDFTLSISCLQSEDFATYYC | |
| | JS140 | SQFSGRGPGADFTLTISSLQPEDFATYYC | |
| | JS40-1, JS48 | SQFSGRGPGTDFTLTISSLQPEDFATYYC | |
| | JS9 | PERFSGSNTGNTATLTITGTQAMDEADYY | |
| | | C | |
| | JS30 | SQFSGSGPGTDFTLTIGSLQPEGFATYYC | |
| | JS41 | SQFRGSGPGTDFTLTISSLQPEDFATYYC | |
| | JS19 | DRFSGSKSGTSASLAISGLQSEDEADYYC | |
| FR4 | JS116, JS144, JS48-1, JS40 | FGGGTKLSVLG | |
| | JS140, JS40-1, JS30, JS41, JS48 | FGGGTKVDIKR | |
| | JS9 | FGGGTKLTVLG | |
| | JS19 | FGTGTKVEIKR | |

| Variant | | | |
|---|---|---|---|
| VH | JS9 | EVQLVGSGGGVVQPGRSLRLSCAAS~GFN FDDHA~~MHWVRQAPGKGLEWVSS~ISG SGGDT~YYADSVKGRFTIARDNSKNTLYL QMNSLRAEDTAVYYC~ATEARGLAH~~~~ ~~WGQGTLVTVSS | |
| | | SSELTQDPAVSVALGQTASITCSGH~~KLGE RYACWY~~~QQKPGQSPVLVIY~~~QDTKR PSGI~~~~PERFSGSNTGNTATLTITGTQAM DEADYYC~~~QAWDSSTVV~~FGGGTKLT VLG | |
| VL | | | |
| VH | JS30 | EVQLVESGPGQVKPSETLSLTCSVS~GGSIS SRDW~WGWVRQPPGEGLEWIGE~IYHSG TT~~NYNPSLKSRVTMSMDTSKNQLSLKL TGVTAVDTAVYYC~VTYSASDAAFDS~~~ WGQGTLVTVSS | |
| VL | | ETTLTQSPSSLSASVGDRITITCRAS~QSIST CLNWY~~~QQKPGKAPKSLIY~~~GASILQ SGVS~~~SQFSGSGPGTDFTLTIGSLQPEGF ATYYC~~~QQGNSVPLT~~~FGGGTKVDIK | |
| | | R | |
| VH | JS41 | EVQLVESGPGQVKPSETLSLTCSVS~GGSIS SRDW~WGWVRQPPGEGLEWIGE~IYHSG TT~~NYNPSLKSRVTMSMDTSKNQLSLKL TGVTAVDTAVYYC~VTYSAYDAAFDS~~~ WGQGTLVTVSS | |
| | | ETTLTQSPSSLSASVGDRITITCRAS~~~QSI STYLSWY~~~QQKPGKAPKSLIY~~~GASN LQSGVS~~~SQFRGSGPGTDFTLTISSLQPE DFATYYC~~~QQGNSVPLT~~~FGGGTKVD IKR | |
| VL | | | |
| VH | JS19 | QVQLQESGGGLVQPGGSLRLACAGS~GFT FSNFA~~MAWVRQAPGKGLEWVSG~ISDD GTIT~DYADSVKGRFTISRDNSKNTLYLQM NSLRAEDTAVYYC~VKVPSPAPMQGPDH~ WGQGTTVTVSS | |
| VL | | QSALTQPPSASGTPGQRVTISCSGS~~~SSNI GSNTVNRY~~QQLPGTAPKLLIY~~~SDNQ RPSGVP~~~DRFSGSKSGTSASLAISGLQSE DEADYYC~~AVWDDSLNGPGYVFGTGTK VEIKR | |
| VH | JS40 | QVQLQESGGGLVQPGGSLRLACAGS~GFT FSNFA~~MAWVRLAPGKGLEWVSG~ISDD GTIT~DYADSVKGRFTISRDNSKNTLYLQM NSLRAEDTAVYYC~VKVPSPAPMQGPDY~ WGQGTTVTVSS | |
| VL | | DVVMTQSPSAMSASVGDRVTITCRAS~~Q GISNYLAWF~~~~QQKPGKVPKRLIY~~~A ASSLQSGVP~~~SRFRGRGSGTDFTLSISCL QSEDFATYYC~~~QQYHSYPLT~~~FGGGT KLSVLG | |
| VH | JS48 | QVQLQESGGGLVQPGGSLRLTCAGS~GFT FSNFA~~MAWVRLAPGKGLEWVSG~ISDD GTIT~DYADSVKGRFTISRDNSKNTLYLQM NSLRAEDTAVYYC~VKVPSPAPMQGPDY~ WGQGTLVTVSS | |
| VL | | ETTLTQSPSSLSASVGDRVTITCRAS~~QSIS TYLNWY~~~~QQKPGKAPKSLIY~~~GASN LQSGVS~~~SQFSGRGPGTDFTLTISSLQPE DFATYYC~~~QQGNSVPLT~~~FGGGTKVD IKR | |

### Example 4. Analysis of binding affinity for Fc alpha receptor-ECD region

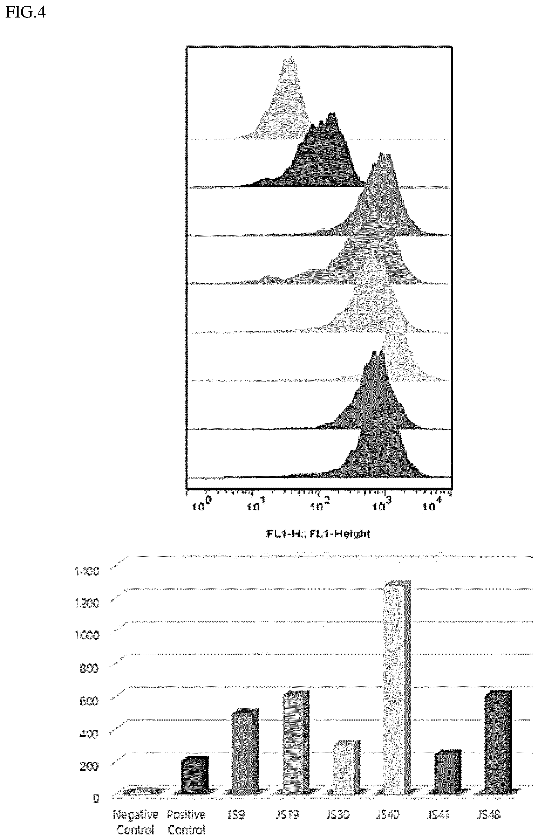

In order to confirm the binding affinity for the Fc alpha receptor-ECD regions of the six scFv antibody variants screened in Example 3, the binding affinity analysis using a flow cytometer was performed. To this end, each variant and Fc (wild type) to be used as a control were prepared into spheroplasts by the method used in Example above. An Fc alpha receptor-ECD-streptavidin-Alexa488 antigen was bound to the prepared spheroplasts at a concentration of 50 nM and incubated at room temperature for 1 hour. Thereafter, in order to remove non-specific binding, the mixture was washed twice with PBS, and the binding affinity for the Fc alpha receptor-ECD region was analyzed using a flow cytometer. As a result, the six scFv variants showed significantly increased fluorescence signals compared to control groups IgG_Fc and IgA_Fc (FIG. 4), and thus, the scFv antibody variants binding to the Fc alpha receptor-ECD region were verified.

### Example 5. Production and purification of antibody IgG including scFv variants

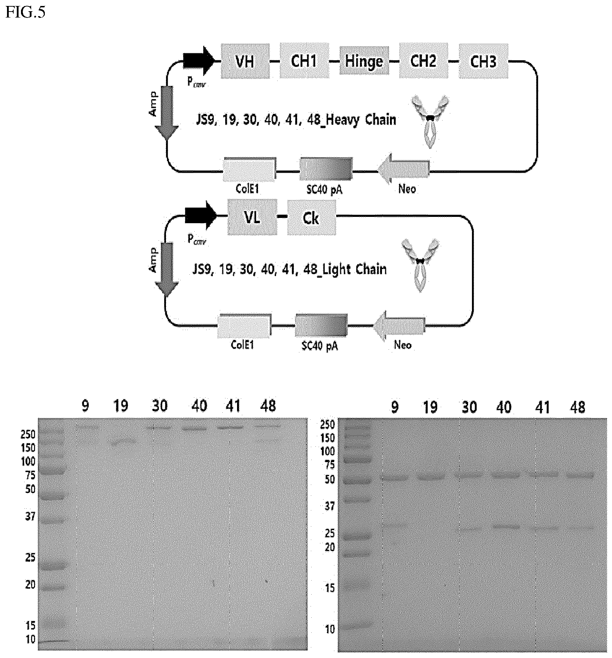

In order to confirm whether the six scFv antibody variants selected in Example 3 had the binding affinity for the Fc alpha receptor even in the form of IgG, heavy chain and light chain expression vectors of the six scFvs were constructed, respectively, and then heavy chain genes and light chain genes of the respective variants were first mixed at a ratio of 1 : 1 in 100 ml of a Freestyle 293 expression culture solution (Gibco, 12338-018), and mixed at a ratio of PEI: variant gene = 4 : 1, left at room temperature for 20 minutes and then transfected into Expi293F cells subcultured the previous day at a density of 2 × 10⁶ cells/ml. Thereafter, the transfected cells were incubated for 7 days under conditions of 37°C, 125 rpm, and 8% CO₂ in a shaking incubator, and then centrifuged to collect only a supernatant. The supernatant was equilibrated with 25x PBS and filtered through a 0.2 µm filter (Merck Millipore) using a bottle top filter. The filtered culture solution was added with 500 µl of Protein A resin and stirred for 16 hours at 4°C to recover the resin through a column, washed with 5 ml PBS, eluted with 3 ml of a 100 mM glycine buffer at pH 2.7, and neutralized using 1 M Tris-HCl pH 8.0. After the buffer was replaced with PBS using Centrifugal filter units 3K (Merck Millipore), the size and purity of IgGs JS9, JS 19, JS30, JS40, JS41 and JS48 containing each of the six purified scFv antibody variants were analyzed by SDS-PAGE (FIG. 5).

### Example 6. Analysis of binding affinity for Fc alpha receptor of antibodies including scFv variants

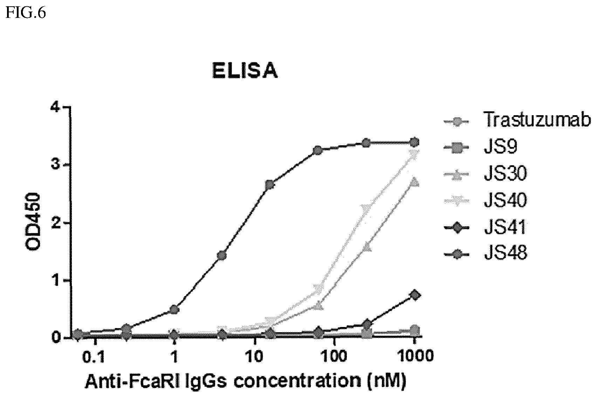

An ELISA experiment was performed to measure the antibody binding affinity for the Fc alpha receptor of the antibodies JS9, JS30, JS40, JS41 and JS48 including the scFv variants prepared in Example 5. Specifically, 50 µl of 4 µg/ml of a tetrameric Fc alpha receptor (antigen) fused with a streptavidin tag in a 0.05 M Na₂CO₃ solution at pH 9.6 was immobilized in a Flat Bottom Polystyrene High Bind 96-well microplate (costar) for 16 hours at 4°C and then blocked at room temperature for 2 hours with 100 µl of 4% skim milk (GenomicBase) (in 0.05% PBST pH 6.0/pH 7.4). After washing four times with 180 µl of 0.05% PBST, 50 µl of antibodies JS9, JS30, JS40, JS41 and JS48 serially diluted with 1% skim milk (in 0.05% PBST) were dispensed into each well and reacted at room temperature for 1 hour. After the washing process, the antibody reaction was performed with 50 µl of anti-human IgG (H+L)-HRP conjugate (Jackson Immunoresearch) at room temperature for 1 hour, respectively, and then washed. Thereafter, 50 µl of a 1-Step Ultra TMB-ELISA Substrate Solution (Thermo Fisher Scientific) was added to develop the color, and then the reaction was terminated by adding 50 µl of 2 M H₂SO₄ and then the binding affinity for the Fc alpha receptor as the antigen of each antibody was analyzed using an Epoch Microplate Spectrophotometer (BioTek) (FIG. 6).

### Example 7. Screening of human antibodies with improved binding affinity for Fc alpha receptor-ECD region using high-speed search system

An experiment was designed to increase the binding affinity for the Fc alpha receptor using two antibodies JS40 and JS48 whose binding affinity to the Fc alpha receptor was confirmed as parent antibodies. To this end, a library containing random mutations at 0.2% was constructed through error prone PCR on JS40 and JS48 antibodies displayed as scFv. In addition, human antibodies binding to the Fc alpha receptors were screened using the Fc alpha receptor-ECD-Streptavidn-Alexa488 probe as an antigen which was used for primary antibody discovery, and the human scFv antibody Error-prone library displayed in bacteria. 1 ml of the library cells were incubated for 4 hours at 37°C and 250 rpm in 25 ml of a TB medium containing 2% glucose and 40 µg/ml chloramphenicol. The incubated cells were cultured in 100 ml of the TB medium containing 40 µg/ml of chloramphenicol at a ratio of 1 : 100 to be OD₆₀₀ = 0.5, and then cooled at 25°C and 250 rpm for 20 minutes, added with 1 mM IPTG, and induced at 25°C and 250 rpm for 5 hours to obtain E. coli overexpressing the protein based on OD₆₀₀ = 8. In order to make the cells into spheroplasts suitable for screening using the flow cytometer, the cells were resuspended using 1 ml of 10 Mm Tris-HCl (pH 8.0) and washed twice to remove the remaining medium, and then rotated in 1 ml of a STE [0.5 M sucrose, 10 Mm Tris-HCl, and 10 Mm EDTA (pH 8.0)] solution at 37°C for 30 minutes to remove the cell outer membrane by osmotic shock. The cells were rotated in a mixed solution of 1 ml of Solution A and 20 µl of 50 mg/ml lysozyme solution at 37°C for 15 minutes to remove a peptidoglycan layer, and washed with 1 ml of PBS, and then 300 µl of the cells were added with 700 µl of PBS and 200 nM (based on monomer) of an Fc alpha receptor-ECD-streptavidin-Alexa488 probe and rotated at room temperature for 1 hour to label spheroplasts with fluorescent probes. Clones showing high fluorescence due to increased binding affinity of antigen were recovered using the flow cytometer (FIG. 2), and in the recovered clones, processes of amplifying scFv genes by PCR, culture, expression induction, spheroplasting to remove a cell outer membrane and a peptidoglycan layer, and selecting spheroplasts showing high affinity in the Fc alpha receptor-ECD region through selective gating of antigen labeling and flow cytometry were repeated. After the selection process using the flow cytometer and the re-selection process to increase the selection purity, an enrichment process, a process of securing the selected scFv variant gene through PCR amplification, a subcloning process, and a transformation process were performed, and the next round of selection was performed to enrich desired clones. Through such a repeated search process at several rounds, novel scFv variant clones with excellent binding affinity to the Fc alpha receptor-ECD region were secured.

### Example 8. Identification of gene sequences of antibody variants with increased binding affinity for Fc alpha receptor

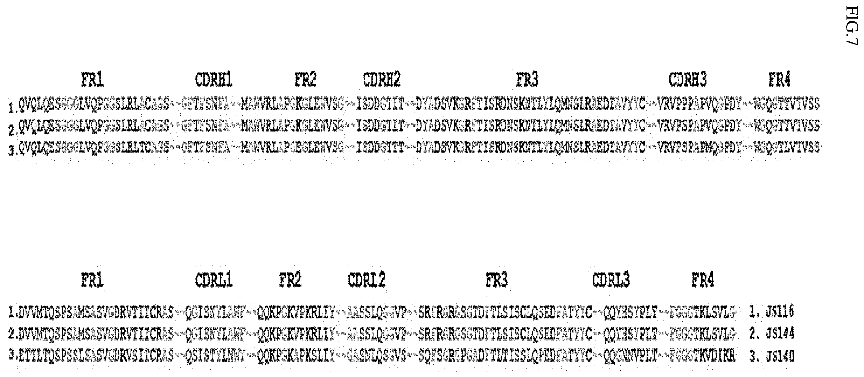

In order to identify the gene sequences of the scFv variant clones obtained in Example 7, DNA sequencing was analyzed using Sanger sequencing (Table 1). Through the analysis, three scFv antibody variants JS 116, JS144 and JS140 having antibody sequences were selected (FIG. 7).

### Example 9. Analysis of binding affinity for ECD region of antibody variants with increased binding affinity for Fc alpha receptor

In order to confirm the binding affinity for the Fc alpha receptor-ECD regions of the selected three scFv antibody variants JS116, JS144 and JS140, the binding affinity analysis using a flow cytometer was performed. To this end, each variant and wild-type Fc to be used as a control were prepared as spheroplasts by the method described in Example 7 above, respectively. The Fc alpha receptor-ECD-streptavidin-Alexa488 antigen used for screening was bound to the prepared spheroplasts at a concentration of 50 nM and incubated at room temperature for 1 hour. In order to remove non-specific binding, the mixture was washed twice with PBS, and the binding affinity for the Fc alpha receptor-ECD region was analyzed using a flow cytometer.

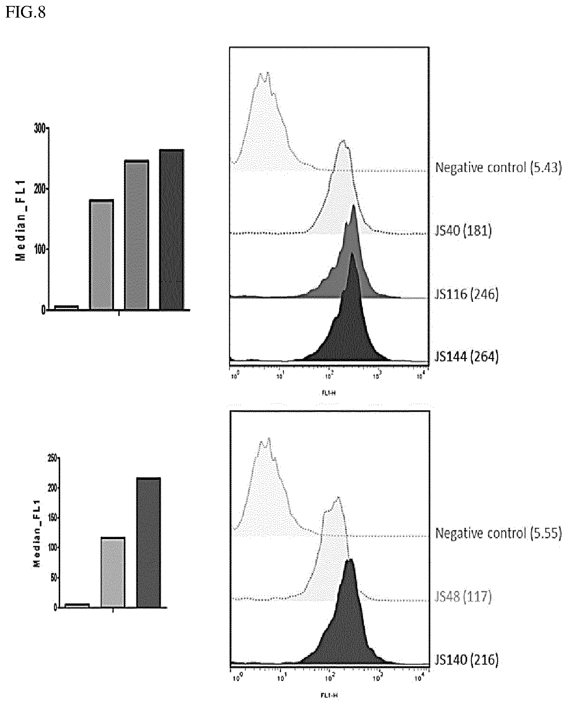

As a result, all of the three scFv variants JS116, JS144 and JS140 showed increased fluorescence signals than the existing control JS40 or JS48 (FIG. 8), and thus, it was confirmed that the scFv antibody variants discovered in the present invention were variants with increased binding affinity for the Fc alpha receptor-ECD region.

### Example 10. Antibody production and purification of three scFv variants with increased binding affinity for Fc alpha receptor-ECD region

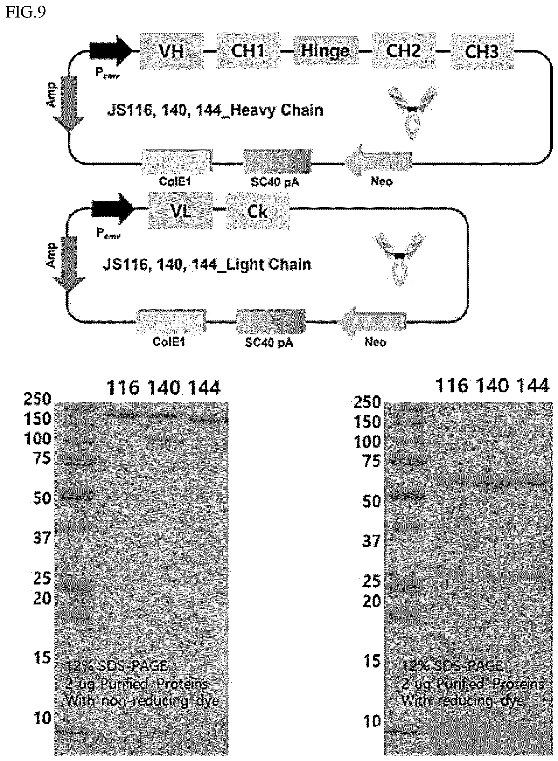

In order to confirm whether the three scFv variants JS116, JS144 and JS 140 selected in Example increased the binding affinity for the Fc alpha receptor even in the form of IgG, heavy chain and light chain expression vectors of the three scFvs were constructed, respectively, and then heavy chain genes and light chain genes of the respective variants were first mixed at a ratio of 1 : 1 in 100 ml of a Freestyle 293 expression culture solution (Gibco, 12338-018), and mixed at a ratio of PE I: variant gene = 4 : 1, left at room temperature for 20 minutes and then transfected into Expi293F cells subcultured the previous day at a density of 2 × 10⁶ cells/ml. Thereafter, the transfected cells were incubated for 7 days under conditions of 37°C, 125 rpm, and 8% CO₂ in a shaking incubator, and then centrifuged to collect only a supernatant. The supernatant was equilibrated with 25x PBS and filtered through a 0.2 µm filter (Merck Millipore) using a bottle top filter. The filtered culture solution was added with 500 µl of Protein A resin and stirred for 16 hours at 4°C to recover the resin through a column, washed with 5 ml PBS, eluted with 3 ml of a 100 mM glycine buffer at pH 2.7, and neutralized using 1 M Tris-HCl pH 8.0. After the buffer was replaced with PBS using Centrifugal filter units 3K (Merck Millipore), the size and purity of IgGs containing each of the three purified scFv antibody variants were analyzed by SDS-PAGE (FIG. 9).

### Example 11. Analysis of binding affinity of antibodies with increased binding affinity for Fc alpha receptor

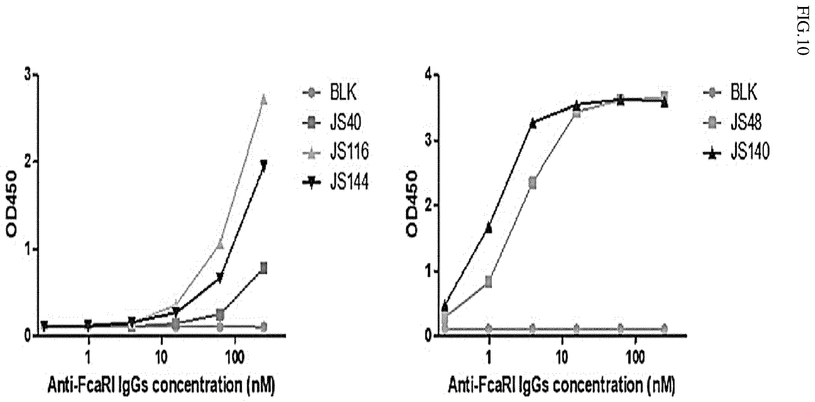

An ELISA experiment was performed to confirm the binding affinity for the Fc alpha receptor on the protein of the antibodies prepared in Example 10. Specifically, 50 µl of 4 µg/ml of a tetrameric Fc alpha receptor (antigen) fused with a streptavidin tag in a 0.05 M Na₂CO₃ at pH 9.6 was immobilized in a Flat Bottom Polystyrene High Bind 96-well microplate (costar) for 16 hours at 4°C and then blocked at room temperature for 2 hours with 100 µl of 4% skim milk (GenomicBase) (in 0.05% PBST pH 6.0/pH 7.4). After washing four times with 180 µl 0.05% PBST, three antibodies JS116, JS144 and JS140 prepared to include the three scFv variants in Example 10 serially diluted with 1% skim milk (in 0.05% PBST) and two existing antibodies JS40 and JS48 as controls were dispensed into each well by 50 µl and reacted at room temperature for 1 hour. After the washing process, the antibody reaction was performed with 50 µl of anti-human IgG (H+L)-HRP conjugate (Jackson Immunoresearch) at room temperature for 1 hour, respectively, and then washed. Thereafter, 50 µl of a 1-Step Ultra TMB-ELISA Substrate Solution (Thermo Fisher Scientific) was added to develop the color, and then the reaction was terminated by adding 50 µl of 2 M H₂SO₄ and then the binding affinity for the Fc alpha receptor as the antigen of each antibody was analyzed using an Epoch Microplate Spectrophotometer (BioTek) (FIG. 10).

### Example 12. Production and purification of antibodies substituted with VH and VL regions of JS40 and JS48

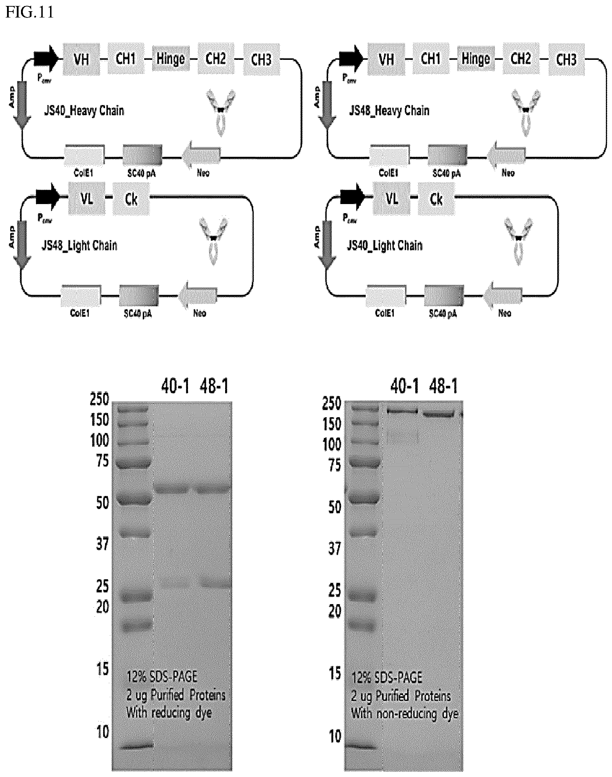

Among the six antibodies discovered in Example 6, it was confirmed that the VH regions of JS40 and JS48, as the two antibodies that showed the highest binding affinity for the Fc alpha receptor on the protein, had very similar sequences, but VLs had completely different sequences, and IgG-type antibodies were prepared by substituting the VH and VL regions of JS40 and JS48, which were used as parent antibodies, with each other (Table 1). To this end, heavy chain and light chain expression vectors of scFvs of a JS40-1 antibody having the VH region of JS40 and the VL region of JS48 and a JS48-1 antibody having the VL region of JS40 and the VH region of JS48 were constructed, respectively, and then heavy chain genes and light chain genes of the respective variants were first mixed at a ratio of 1 : 1 in 100 ml of a Freestyle 293 expression culture solution (Gibco, 12338-018), and mixed at a ratio of PEI : variant gene = 4:1, left at room temperature for 20 minutes and then transfected into Expi293F cells subcultured the previous day at a density of 2 × 10⁶ cells/ml. Thereafter, the transfected cells were incubated for 7 days under conditions of 37°C, 125 rpm, and 8% CO₂ in a shaking incubator, and then centrifuged to collect only a supernatant. The supernatant was equilibrated with 25x PBS and filtered through a 0.2 µm filter (Merck Millipore) using a bottle top filter. The filtered culture solution was added with 500 µl of Protein A resin and stirred for 16 hours at 4°C to recover the resin through a column, washed with 5 ml PBS, eluted with 3 ml of a 100 mM glycine buffer at pH 2.7, and neutralized using 1 M Tris-HCl pH 8.0. After the buffer was replaced with PBS using Centrifugal filter units 3K (Merck Millipore), the size and purity of IgGs containing each of the two purified JS40-1 and JS48-lwere analyzed by SDS-PAGE (FIG. 11).

### Example 13. Analysis of binding affinity for Fc alpha receptor of antibodies JS40-1 and JS48-1

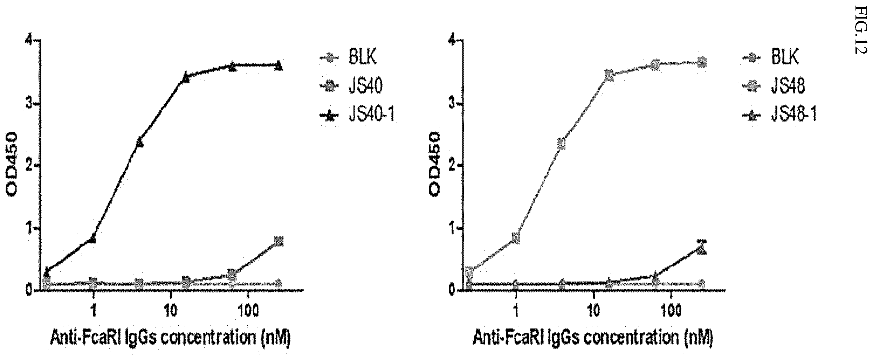

An ELISA experiment was performed to confirm the binding affinity for the Fc alpha receptor on the proteins of the antibodies JS40-1 and JS48-1 prepared in Example 12. Specifically, 50 µl of 4 µg/ml of a tetrameric Fc alpha receptor (antigen) fused with a streptavidin tag in a 0.05 M Na₂CO₃ at pH 9.6 was immobilized in a Flat Bottom Polystyrene High Bind 96-well microplate (costar) for 16 hours at 4°C and then blocked at room temperature for 2 hours with 100 µl of 4% skim milk (GenomicBase) (in 0.05% PBST pH 6.0/pH 7.4). After washing four times with 180 µl 0.05% PBST, the two antibodies JS40-1 and JS48-1 prepared in Example 12 serially diluted with 1% skim milk (in 0.05% PBST) and two existing antibodies JS40 and JS48 as controls were dispensed into each well by 50 µl and reacted at room temperature for 1 hour. After the washing process, the antibody reaction was performed with 50 µl of anti-human IgG (H+L)-HRP conjugate (Jackson Immunoresearch) at room temperature for 1 hour, respectively, and then washed. Thereafter, 50 µl of a 1-Step Ultra TMB-ELISA Substrate Solution (Thermo Fisher Scientific) was added to develop the color, and then the reaction was terminated by adding 50 µl of 2 M H₂SO₄ and then the binding affinity for the Fc alpha receptor as the antigen of each antibody was analyzed using an Epoch Microplate Spectrophotometer (BioTek) (FIG. 12).

### Example 14. Production and purification of bispecific antibody

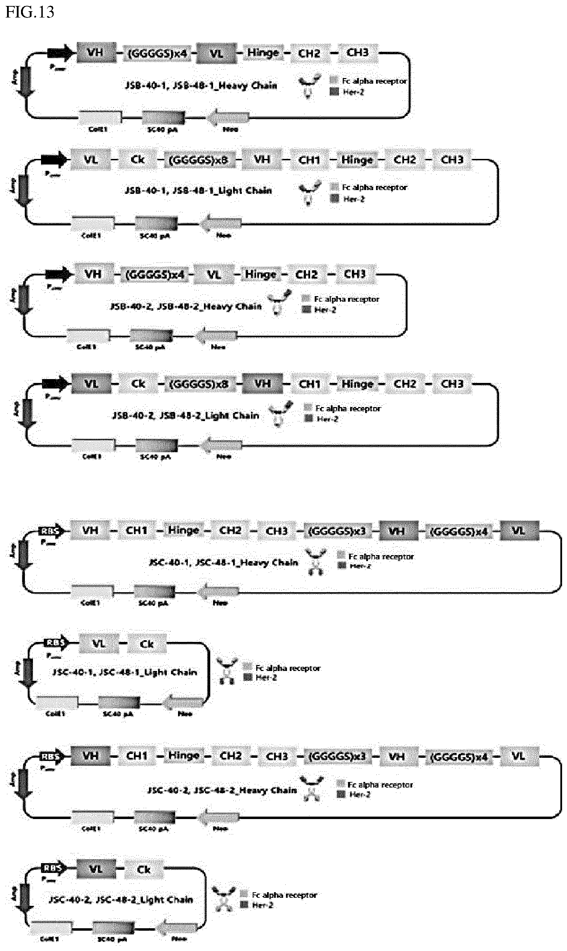

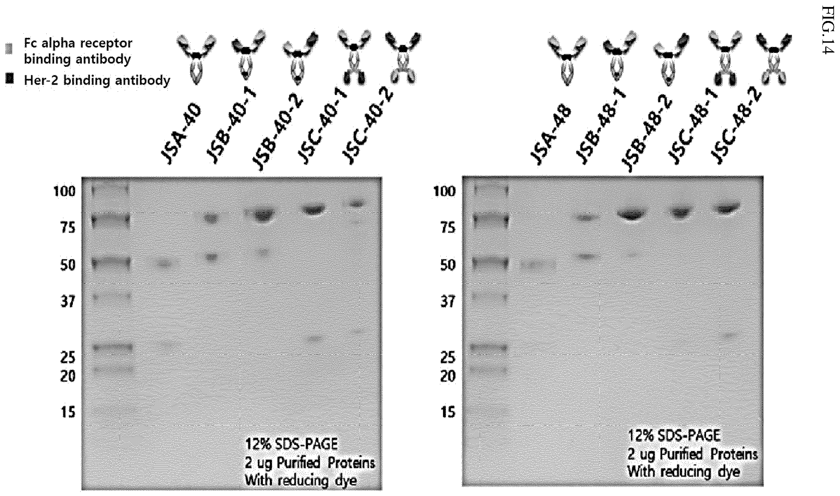

In order to produce a therapeutic antibody that maximized an effector function of neutrophils using antibodies binding to the Fc alpha receptor, in Example 13, the antibodies JS40 and JS48, which had high binding affinity for the Fc alpha receptor, were prepared in the form of bispecific antibodies, respectively. Specifically, heavy chain and light chain expression vectors of eight antibodies were constructed, respectively, to prepare four types of bispecific antibodies, JSB-40-1 (JS40 Fab + scFv of Trastuzumab), JSB-48-1 (JS48 Fab + scFv of Trastuzumab), JSB-40-2 (JS40 scFv + Fab of Trastuzumab) and JSB-48-2 (JS48 scFv + Trastuzumab Fab), which were introduced with a Knob T366W and Holes T366S, L368A, Y407V into Fc, in the form of scFv and Fab of Trastuzumab targeting JS40, JS48, and Her-2, respectively, and to prepare four types of bispecific antibodies, JSC-40-1 (JS40 IgG + Trastuzumab scFv), JSC-48-1 (JS48 IgG + Trastuzumab scFv), JSC-40-2 (Trastuzumab IgG + JS40 scFv) and JSC-48-2 (Trastuzumab IgG + JS48 scFv), in which JS40, JS48 and trastuzumab were linked to the Fc terminus of each antibody in the scFv form (FIG. 13). Then, in JSB-40-1, JSB-40-2, JSB-48-1, and JSB-48-2, which expressed and purified the antibodies as in Example, the size and purity of anti-CK resin, JSC-40-1, JSC-40-2, JSC-48-1 and JSC-48-2 were analyzed by SDS-PAGE (FIG. 14).

### Example 15. Analysis of binding affinity for two antigens of bispecific antibody

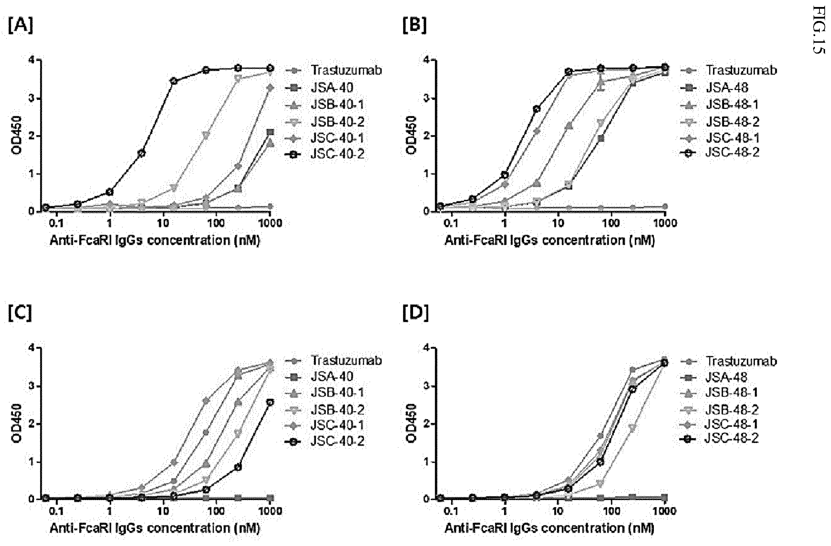

In order to measure the binding affinity for two antigens, Fc alpha receptor and Her-2 of the bispecific antibody prepared in Example 14, ELISA analysis was performed under the same conditions as in the previous experiment. Specifically, the bispecific antibodies were reacted by serial dilution, washed, and then antibody-reacted with 50 µl of a Protein L-HRP conjugate (GenScript) at room temperature for 1 hour, and washed. Thereafter, 50 µl of a 1-Step Ultra TMB-ELISA Substrate Solution (Thermo Fisher Scientific) was added to develop the color, and then the reaction was terminated by adding 50 µl of 2 M H₂SO₄ and then the binding affinity for the Fc alpha receptor as the antigen of each antibody was analyzed using an Epoch Microplate Spectrophotometer (BioTek). As a result, all of the bispecific antibodies showed excellent binding affinity for the Her-2 antigen and the Fc alpha receptor, and various bispecific antibody formats differed in binding affinity, but specifically bound to the two antigens (FIG. 15).

## Claims

1. An antibody specific for an Fc alpha receptor or an immunologically active fragment thereof comprising:
(a) a VH domain including complementarity determining region heavy chain (CDRH)1 including any one selected from the group consisting of amino acid sequences of SEQ ID NOs: 1 to 3, CDRH2 including any one selected from the group consisting of amino acid sequences of SEQ ID NOs: 4 to 7, and CDRH3 including any one selected from the group consisting of amino acid sequences of SEQ ID NOs: 8 to 15; and/or
(b) a VL domain including complementarity determining region light chain (CDRL)1 including any one selected from the group consisting of amino acid sequences of SEQ ID NOs: 16 to 21, CDRL2 including any one selected from the group consisting of amino acid sequences of SEQ ID NOs: 22 to 27, and CDRL3 including any one selected from the group consisting of amino acid sequences of SEQ ID NOs: 28 to 32.

2. The antibody specific for the Fc alpha receptor or the immunologically active fragment thereof of claim 1, comprising: (a) a VH domain including FR1 including any one selected from the group consisting of amino acid sequences of SEQ ID NOs: 33 to 36, FR2 including any one selected from the group consisting of amino acid sequences of SEQ ID NOs: 37 to 41, FR3 including any one selected from the group consisting of amino acid sequences of SEQ ID NOs: 42 to 44, and FR4 including an amino acid sequence of SEQ ID NO: 45 or 46; and/or
(b) a VL domain including FR1 including any one selected from the group consisting of amino acid sequences of SEQ ID NOs: 47 to 52, FR2 including any one selected from the group consisting of amino acid sequences of SEQ ID NOs: 53 to 56, FR3 including any one selected from the group consisting of amino acid sequences of SEQ ID NOs: 57 to 63, and FR4 including any one selected from the group consisting of amino acid sequences of SEQ ID NOs: 64 to 67.

3. The antibody specific for the Fc alpha receptor or the immunologically active fragment thereof of claim 1, comprising: a V domain including (a) a VH domain including CDRH1 including an amino acid sequence of SEQ ID NO: 1, CDRH2 including an amino acid sequence of SEQ ID NO: 4, and CDRH3 including an amino acid sequence of SEQ ID NO: 11; and/or
(b) a VL domain including CDRL1 including an amino acid sequence of SEQ ID NO: 16, CDRL2 including an amino acid sequence of SEQ ID NO: 24, and CDRL3 including an amino acid sequence of SEQ ID NO: 28.

4. The antibody specific for the Fc alpha receptor or the immunologically active fragment thereof of claim 1, comprising: a V domain including (a) a VH domain including CDRH1 including an amino acid sequence of SEQ ID NO: 1, CDRH2 including an amino acid sequence of SEQ ID NO: 4, and CDRH3 including an amino acid sequence of SEQ ID NO: 11; and/or
(b) a VL domain including CDRL1 including an amino acid sequence of SEQ ID NO: 17, CDRL2 including an amino acid sequence of SEQ ID NO: 23, and CDRL3 including an amino acid sequence of SEQ ID NO: 30.

5. The antibody specific for the Fc alpha receptor or the immunologically active fragment thereof of claim 1, comprising: a V domain including (a) a VH domain including CDRH1 including an amino acid sequence of SEQ ID NO: 1, CDRH2 including an amino acid sequence of SEQ ID NO: 4, and CDRH3 including an amino acid sequence of SEQ ID NO: 8; and/or
(b) a VL domain including CDRL1 including an amino acid sequence of SEQ ID NO: 16, CDRL2 including an amino acid sequence of SEQ ID NO: 22, and CDRL3 including an amino acid sequence of SEQ ID NO: 28.

6. The antibody specific for the Fc alpha receptor or the immunologically active fragment thereof of claim 1, comprising: a V domain including (a) a VH domain including CDRH1 including an amino acid sequence of SEQ ID NO: 1, CDRH2 including an amino acid sequence of SEQ ID NO: 4, and CDRH3 including an amino acid sequence of SEQ ID NO: 9; and/or
(b) a VL domain including CDRL1 including an amino acid sequence of SEQ ID NO: 16, CDRL2 including an amino acid sequence of SEQ ID NO: 22, and CDRL3 including an amino acid sequence of SEQ ID NO: 28.

7. The antibody specific for the Fc alpha receptor or the immunologically active fragment thereof of claim 1, comprising: a V domain including (a) a VH domain including CDRH1 including an amino acid sequence of SEQ ID NO: 1, CDRH2 including an amino acid sequence of SEQ ID NO: 4, and CDRH3 including an amino acid sequence of SEQ ID NO: 10; and/or
(b) a VL domain including CDRL1 including an amino acid sequence of SEQ ID NO: 17, CDRL2 including an amino acid sequence of SEQ ID NO: 23, and CDRL3 including an amino acid sequence of SEQ ID NO: 29.

8. The antibody specific for the Fc alpha receptor or the immunologically active fragment thereof of claim 1, comprising: a V domain including (a) a VH domain including FR1 including an amino acid sequence of SEQ ID NO: 34, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 11, and FR4 including an amino acid sequence of SEQ ID NO: 46; and/or
(b) a VL domain including FR1 including an amino acid sequence of SEQ ID NO: 49, CDRL1 including an amino acid sequence of SEQ ID NO: 16, FR2 including an amino acid sequence of SEQ ID NO: 54, CDRL2 including an amino acid sequence of SEQ ID NO: 24, FR3 including an amino acid sequence of SEQ ID NO: 59, CDRL3 including an amino acid sequence of SEQ ID NO: 28, and FR4 including an amino acid sequence of SEQ ID NO: 65.

9. The antibody specific for the Fc alpha receptor or the immunologically active fragment thereof of claim 1, comprising: a V domain including (a) a VH domain including FR1 including an amino acid sequence of SEQ ID NO: 33, CDRH1 including an amino acid sequence of SEQ ID NO: 1, FR2 including an amino acid sequence of SEQ ID NO: 37, CDRH2 including an amino acid sequence of SEQ ID NO: 4, FR3 including an amino acid sequence of SEQ ID NO: 42, CDRH3 including an amino acid sequence of SEQ ID NO: 11, and FR4 including an amino acid sequence of SEQ ID NO: 45; and/or
(b) a VL domain including FR1 including an amino acid sequence of SEQ ID NO: 47, CDRL1 including an amino acid sequence of SEQ ID NO: 17, FR2 including an amino acid sequence of SEQ ID NO: 53, CDRL2 including an amino acid sequence of SEQ ID NO: 23, FR3 including an amino acid sequence of SEQ ID NO: 57, CDRL3 including an amino acid sequence of SEQ ID NO: 30, and FR4 including an amino acid sequence of SEQ ID NO: 64.

10. The antibody specific for the Fc alpha receptor or the immunologically active fragment thereof of claim 1, comprising: a V domain including (a) a VH domain including CDRH1 including an amino acid sequence of SEQ ID NO: 2, CDRH2 including an amino acid sequence of SEQ ID NO: 6, and CDRH3 including an amino acid sequence of SEQ ID NO: 12; and/or (b) a VL domain including CDRL1 including an amino acid sequence of SEQ ID NO: 18, CDRL2 including an amino acid sequence of SEQ ID NO: 25, and CDRL3 including an amino acid sequence of SEQ ID NO: 31.

11. The antibody specific for the Fc alpha receptor or the immunologically active fragment thereof of claim 1, comprising: a V domain including (a) a VH domain including CDRH1 including an amino acid sequence of SEQ ID NO: 1, CDRH2 including an amino acid sequence of SEQ ID NO: 4, and CDRH3 including an amino acid sequence of SEQ ID NO: 15; and/or
(b) a VL domain including CDRL1 including an amino acid sequence of SEQ ID NO: 21, CDRL2 including an amino acid sequence of SEQ ID NO: 27, and CDRL3 including an amino acid sequence of SEQ ID NO: 32.

12. The antibody specific for the Fc alpha receptor or the immunologically active fragment thereof of claim 1, comprising: a V domain including (a) a VH domain including CDRH1 including an amino acid sequence of SEQ ID NO: 3, CDRH2 including an amino acid sequence of SEQ ID NO: 7, and CDRH3 including an amino acid sequence of SEQ ID NO: 13; and/or
(b) a VL domain including CDRL1 including an amino acid sequence of SEQ ID NO: 19, CDRL2 including an amino acid sequence of SEQ ID NO: 26, and CDRL3 including an amino acid sequence of SEQ ID NO: 30.

13. The antibody specific for the Fc alpha receptor or the immunologically active fragment thereof of claim 1, comprising: a V domain including (a) a VH domain including CDRH1 including an amino acid sequence of SEQ ID NO: 3, CDRH2 including an amino acid sequence of SEQ ID NO: 7, and CDRH3 including an amino acid sequence of SEQ ID NO: 14; and/or
(b) a VL domain including CDRL1 including an amino acid sequence of SEQ ID NO: 20, CDRL2 including an amino acid sequence of SEQ ID NO: 23, and CDRL3 including an amino acid sequence of SEQ ID NO: 30.

14. The antibody specific for the Fc alpha receptor or the immunologically active fragment thereof of claim 1, wherein the immunologically active fragment is any one selected from the group consisting of Fab, Fd, Fab', dAb, F(ab'), F(ab')₂, a single chain fragment variable (scFv), Fv, a single chain antibody, a Fv dimer, a complementarity determining region fragment, a humanized antibody, a chimeric antibody, and a diabody.

15. A bispecific or multispecific antibody comprising the antibody or the immunologically active fragment thereof of claim 1, and a moiety binding to a target antigen other than the Fc alpha receptor.

16. The bispecific or multispecific antibody of claim 15, wherein the moiety binding to the target antigen comprises an antibody or an immunologically active fragment thereof.

17. The bispecific or multispecific antibody of claim 15, wherein the target antigen is at least one selected from the group consisting of 17-1A antigen, GD3 ganglioside R24, EGFRvIII, PSMA, PSCA, HLA-DR, EpCAM, MUC1 core protein, aberrant glycosylation MUC1, fibronectin heteroform containing ED-B domain, HER2/neu, carcinoembryonic antigen (CEA), gastrin-releasing peptide (GRP) receptor antigen, mucine antigen, epidermal growth factor receptor (EGF-R), HER3, HER4, MAGE antigen, SART antigen, MUC1 antigen, c-erb-2 antigen, TAG 72, carbonic anhydrase IX, alpha-fetoprotein, A3, an antigen specific to an A33 antibody, Ba 733, BrE3-antigen, CA125, CDl, CD1a, CD3, CD5, CD15, CD16, CD19, CD20, CD21, CD22, CD23, CD25, CD30, CD33, CD38, CD40, CD45, CD52, CD74, CD79a, CD80, CD138, colon-specific antigen-p (CSAp), CSAp, EGP-1, EGP-2, Ep-CAM, FIt-1, Flt-3, folate receptor, HLA-DR, human chorionic gonadotropin (HCG) and its subunits, hypoxia inducible factor (HIF-I), Ia, IL-2, IL-6, IL-8, insulin growth factor-1 (IGF-1), KC4-antigen, KS-1-antigen, KS1-4, Le-Y, macrophage inhibitory factor (MIF), MAGE, MUC1, MUC2, MUC3, MUC4, NCA66, NCA95, NCA90, antigen specific for PAM-4 antibody, placental growth factor, p53, prostatic acid phosphatase, PSA, RS5, S1OO, TAC, tenascin, TRAIL receptors, Tn antigen, Thomson-Friedenreich antigens, tumor necrosis antigens, VEGF, ED-B fibronectin, angiogenesis marker, oncogene marker or oncogene product.

18. The bispecific or multispecific antibody of claim 17, wherein the oncogene is at least one selected from the group consisting of SEPTIN9, ACOD1, ACTN4, ADAM28, ADAM9, ADGRF1, ADRBK2, AFF1, AFF3, AGAP2, AGFG1, AGRN, AHCYL1, AHI1, AIMP2, AKAP13, AKAP9, AKIRIN2, AKTIP, ALDH1A1, ALL1, ANIB1, ANP32C, ANP32D, AQP1, ARAF, ARHGEF1, ARHGEF2, ARHGEF5, ASPSCR1, AURKA, BAALC, BAIAP2L1, BANP, BCAR4, BCKDHB, BCL9, BCL9L, BCR, BMI1, BMP7, BOC, BRD4, BRF2, CABIN1, CAMK1D, CAPG, CBFB, CBLB, CBLL1, CBX7, CBX8, CCDC28A, CCDC6, CCNB1, CCNB2, CCND1, CCNE1, CCNL1, CD24, CDC25C, CDC6, CDH17, CDK1, CDK14, CDK4, CDK5R2, CDK6, CDK8, CDKN1B, CDKN3, CDON, CEACAM6, CENPW, CHD1L, CHIC1, CHL1, CKS1B, CMC4, CNTN2, COPS3, COPS5, CRKL, CRLF2, CROT, CRTC1, CRYAB, CSF1R, CSF3, CSF3R, CSNK2A1, CSNK2A2, CT45A1, CTBP2, CTNND2, CTSZ, CUL7, CXCL1, CXCL2, CXCL3, CYGB, CYP24A1, DCD, DCUN1D1DDB2, DDHD2, DDX6, DEK, DIS3, DNPH1, DPPA2, DPPA4, DSG3, DUSP12, DUSP26, ECHS1, ECT2, EEF1A1, EEF1A2, EEF1D, EIF3E, EIF3I, EIF4E, EIF5A2, ELAVL1, ELL, EML4, EMSY, ENTPD5, EPCAM, EPS8, ERAS, ERGIC1, ERVW-1, EVI2A, EVI5, EWSR1, EZH2, FAM189B, FAM72A, FAM83D, FASN, FDPS, FGF10, FGF3, FGF5, FGF8, FR1OP, FHL2, FIP1L1, FNDC3B, FRAT1, FUBP1, FUS, FZD2, GAB2, GAEC1, GALNT10, GALR2, GLO1, GMNN, GNA12, GNA13, GNAI2, GNAQ, GNAS, GOLPH3, GOPC, GPAT4, GPM6A, GPM6B, GPR132, GREM1, GRM1, GSK3A, GSM1, H19, HAS1, HAX1, HDGFRP2, HMGN5, HNRNPA1, HOTAIR, HOTTIP, HOXA-AS2, HRAS, HSPA1A, HSPA4, HSPB1, HULC, IDH1, IFNG, IGF2BP1, IKBKE, IL7R, INPPL1, INTS1, INTS2, INTS3, INTS4, INTS5, INTS7, INTS8, IRS2, IST1, JUP, KDM4C, KIAA0101, KIAA1524, KIF14, KRAS, KSR2, LAMTOR5, LAPTM4B, LCN2, LDHB, LETMD1, LIN28A, LIN28B, LMO1, LMO2, LMO3, LMO4, LSM1, LUADT1, MACC1, MACROD1, MAGEA11, MALAT1, MAML2, MAP3K8, MAPRE1, MAS1, MCC, MCF2, MCF2L, MCTS1, MEFV, MFHAS1, MFNG, MIEN1, MINA, MKL2, MLANA, MLLT1, MLLT11, MLLT3, MLLT4, MMP12, MMS22L, MN1, MNAT1, MOS, MPL, MPST, MRAS, MRE11A, MSI1, MTCP1, MTDH, MTOR, MUC1, MUC4, MUM1, MYD88, NAAA, NANOGP8, NBPF12, NCOA4, NEAT1, NECTIN4, NEDD4, NEDD9, NET1, NINL, NME1, NOTCH1, NOTCH4, NOV, NSD1, NUAK2, NUP214, NUP98, NUTM1, OLR1, PA2G4, PADI2, PAK7, PARK7, PARM1, PBK, PCAT1, PCAT5, PDGFA, PDZK1IP1, PELP1, PFN1P3, PIGU, PIK3CA, PIK3R1, PIM1, PIM2, PIM3, PIR, PIWIL1, PLAC8, PLK1, PPM1D, PPP1R10, PPP1R14A, PPP2R1A, PRAME, PRDM12, PRMT5, PSIP1, PSMD10, PTCH2, PTMA, PTP4A1, PTP4A2, PTP4A3, PTTG1, PTTG1IP, PTTG2, PVT1, RAB11A, RAB18, RAB22A, RAB23, RAB8A, RALGDS, RAP1A, RASSF1, RBM14, RBM15, RBM3, RBMY1A1, RFC3, RGL4, RGR, RHO, RING1, RINT1, RIT1, RNF43, RPL23, RRAS, RRAS2, RSF1, RUNX1T1, S100A4, S100A7, S100A8, SAG, SART3, SBSN, SEA, SEC62, SERTAD1, SERTAD2, SERTAD3, SET, SETBP1, SETDB1, SGK1, SIRT1, SIRT6, SKI, SKIL, SKP2, SLC12A5, SLC3A2, SMR3B, SMURF1, SNCG, SNORA59A, SNORA80E, SPAG9, SPATA4, SPRY2, SQSTM1, SRSF1, SRSF2, SRSF3, SRSF6, SS18, SSX1, SSX2, SSX2B, STIL, STMN1, STRA6, STYK1, SUZ12, SWAP70, SYT1, TAC1, TACSTD2, TAF15, TALDO1, TAZ, TBC1D1, TBC1D15, TBC1D3, TBC1D3C, TBC1D7, TCL1A, TCL1B, TCL6, TCP1, TFG, TGM3, TINCR, TKTL1, TLE1, TMEM140, TMPOP2, TMPRSS2, TNS4, TPD52, TPR, TRE17, TREH, TRIB1, TRIB2, TRIM28, TRIM32, TRIM8, TRIO, TRIP6, TSPAN1, TSPY1, TXN, TYMS, TYRP1, UBE2C, UBE3C, UCA1, UCHL1, UHRF1, URI1, USP22, USP4, USP6, VAV1, VAV2, VAV3, VIM, WAPL, WHSC1, WHSC1L1, WISP1, WNT1, WNT10A, WNT10B, WNT2, WNT3, WNT5A, WWTR1, XCL1, XIAP, YAP1, YEATS4, YY1AP1, ZEB1-AS1, ZFAND4, ZFAS1, ZMYM2, ZNF703 and ZNHIT6.

19. The bispecific or multispecific antibody of claim 15, wherein the target antigen is a cell surface antigen.

20. The bispecific or multispecific antibody of claim 19, wherein the cell surface antigen is at least one selected from the group consisting of CEA, ED-B fibronectin, CD20, CD22, CD19, EGFR, IGF1R, VEFGRl/Flt-1, VEGFR2/KDR, VEGRF3/Flt-4, HER2/neu, CD30, CD33, CD3, CD16, CD64, CD89, CD2, adenovirus fiber knob, PfMSP-1, HN/NDV, EpCAM/17-lA, hTR, IL-2R/Tac, CA19-9, MUC1, HLA class II, GD2, G250, TAG-72, PSMA, CEACAM6, HMWMAA, CD40, M13 enveloped protein and GPIIb/IIIa.

21. The bispecific or multispecific antibody of claim 15, wherein the target antigen is an autoantigen.

22. An isolated nucleic acid molecule encoding the antibody or the immunologically active fragment thereof of claim 1 or the bispecific or multispecific antibody of claim 15.

23. A vector comprising the isolated nucleic acid molecule of claim 22.

24. A host cell transformed with the vector of claim 23.

25. A pharmaceutical composition comprising the bispecific or multispecific antibody of claim 15 as an active ingredient for prevention or treatment of a disease or disorder selected from the group consisting of cancer, autoimmune disease, neurodegerative disease, Alzheimer's disease, metabolic disease, cardiovascular disease, atherosclerosis, organ transplant rejection, and diseases or symptoms caused by fungi, viruses, bacteria or parasites.

26. A method for producing an antibody specific for an Fc alpha receptor or an immunologically active fragment thereof comprising:
a) culturing a host cell transformed with a vector including an isolated nucleic acid molecule encoding the antibody or the immunologically active fragment thereof of claim 1; and
b) recovering the antibody or the immunologically active fragment thereof from the host cell culture.

27. A method for producing a bispecific or multispecific antibody binding to an Fc alpha receptor and a target antigen other than the Fc alpha receptor comprising:
a) culturing a host cell transformed with a vector including an isolated nucleic acid molecule encoding the bispecific or multispecific antibody of claim 15; and
b) recovering the antibody or the immunologically active fragment thereof from the host cell culture.

28. A use of an antibody specific for an Fc alpha receptor or an immunologically active fragment thereof for use in the preparation of an antibody therapeutic agent.

29. A use of a bispecific or multispecific antibody for prevention or treatment of a disease or disorder selected from the group consisting of cancer, autoimmune disease, neurodegerative disease, Alzheimer's disease, metabolic disease, cardiovascular disease, atherosclerosis, organ transplant rejection, and diseases or symptoms caused by fungi, viruses, bacteria or parasites.

30. A method for treating cancer comprising administering a bispecific or multispecific antibody in a pharmaceutically effective dose to a subject suffering from cancer.
